# EUROPEAN PATENT SPECIFICATION

(11) **EP 2 649 091 B1**
(45) Date of publication and mention of the grant of the patent: **01.10.2014**
(21) Application number: 11794727.5
(22) Date of filing: 09.12.2011
(51) Int. Cl.: C07K 14/11, C07K 14/285, C07K 14/705, A61K 38/04

(54) **IMMUNE RESTRICTED PEPTIDES WITH INCREASED EFFICACY**
IMMUNBESCHRÄNKTE PEPTIDE MIT VERBESSERTER WIRKUNG
PEPTIDES IMMUNORESTREINTS AVEC EFFICACITÉ AMÉLIORÉE

(30) Priority: 09.12.2010 WO PCT/EP2010/069246
(43) Date of publication of application: 16.10.2013
(73) Proprietor: Stichting Het Nederlands Kanker Instituut, 1066 CX Amsterdam (NL); Stichting Sanquin Bloedvoorziening, 1066 CX Amsterdam (NL)
(72) Inventor: OVAA, Huib, 1066 CX Amsterdam (NL); RODENKO, Boris, 1066 CX Amsterdam (NL); HOPPES, Rieuwert, 1066 CX Amsterdam (NL); AMORE, Alessia, 1066 CX Amsterdam (NL); SCHUMACHER, Antonius, Nicolaas, Maria, 1066 CX Amsterdam (NL)
(74) Representative: van Kooij, Adriaan
(86) International application number: PCT/EP2011/072377
(87) International publication number: WO 2012/076708

(56) References cited:
- B RODENKO ET AL.: "Class I major histocompatibility complexes loaded by a periodate trigger", JOURNAL OF THE AMERICAN CHEMICAL SOCIETY, vol. 131, no. 34, 8 May 2009 (2009-05-08), pages 13205-13213, XP002658373, AMERICAN CHEMICAL SOCIETY ISSN: 0002-7863
- P H N CELIE ET AL.: "UV-induced ligand exchange in MHC class I protein crystals", JOURNAL OF THE AMERICAN CHEMICAL SOCIETY, vol. 131, no. 34, 8 May 2009 (2009-05-08), pages 12298-12304, XP002658374, AMERICAN CHEMICAL SOCIETY ISSN: 0002-7863
- A H BAKKER ET AL.: "Conditional MHC class I ligands and peptide exchange technology for the human MHC gene products HLA-A1, -A11 and -B7", PROCEEDINGS OF THE NATIONAL ACADEMY OF SCIENCES, vol. 105, no. 10, 11 March 2008 (2008-03-11), XP002658375, National Academy of Sciences ISSN: 0027-8424

## Description

The present invention relates to immune restricted peptides, and especially HLA-A2 restricted peptides. Further, the present invention relates to methods for providing the present immune restricted peptides and the use thereof in medicine and especially the use thereof in vaccines, immunosuppressive therapy, adoptive T cell therapy and diagnostics.

Existing and newly emerging diseases that threaten public health demand the development of new technologies combating, or preventing, these diseases. One approach to combat, or prevent, diseases is to use, or direct, the own defence system of a subject, i.e. the immune system, for example by vaccination, immunosuppressive therapy, or adoptive T cell therapy.

A vaccine is a biological preparation that stimulates, activates or improves the response of the immune system towards a particular disease or condition. Vaccines can be prophylactic, for example to prevent or ameliorate the effects of a future infection by a pathogen, or therapeutic, for example vaccines against cancer.

A classical vaccine typically contains an agent that mimics a disease-causing agent such as a microorganism, and is often made from weakened or killed forms of a pathogen.

Besides the above classical vaccines, considerable efforts have been made towards the development of subunit vaccines. Rather than introducing an inactivated or attenuated micro-organism in order to stimulate, activate or improve the immune system, a fragment, such as a peptide, is used.

Peptide vaccines are generally preparations comprising synthetic epitopes in the form of peptides, i.e. short strings of consecutive amino acid forming sequences up to 20, 30, 40 or 50 amino acids, representing one or more minimal immunogenic regions of a protein or antigen.

All nucleated cells present peptides that are derived, or originate, from intracellular proteins on their surface bound to MHC class I, whereas peptides derived, or originate, from extracellular proteins are mainly presented by MHC class II on specialised antigen-presenting cells, APCs, such as dendritic cells and macrophages.

In both cases, the T cell receptor or TCR on the surface of the cytolytic T lymphocyte, CTL, or T_{H} cell forms a complex with the MHC I/peptide-epitope complex or the MHC II/peptide-epitope complex, respectively; these interactions are aided by the CD8 and CD4 co-receptors, respectively. The intricate interplay of these peptide-dependent recognition processes results in the initiation or propagation of immune responses controlling, for example, infections and cancer in a subject, such as a human subject.

Vaccines have been designed based on the use of short synthetic peptides which mimic the exact epitope recognised by cytolytic CD8⁺ T lymphocytes when associated with the restricting MHC complex. This limits the applicability of the vaccine to individuals of the appropriate MHC haplotype. Since HLA alleles are extremely polymorphic, the practical approach to this type of vaccination has focused the efforts on those peptides presented by the most frequent HLA alleles. HLA-A2, and to a lesser extent other alleles such as -A1, -A3, -B7, -B35, are alleles generally relevant for individuals of Caucasian origin.

Despite HLA allele restriction, peptide vaccines offer considerable advantages such as absence of infectious material capable of compromising live or attenuated vaccines. Furthermore, many pathogens can be difficult or impossible to culture by conventional methods. Peptide vaccine also offer the option to exclude deleterious sequences from full-length antigens, such as proteins, or other pathogen-derived molecules such as oncogenic compounds or compounds implicated in autoimmune diseases.

Peptides are easily characterised and analysed for purity using well-established analytical techniques such as liquid chromatography and mass spectrometry. This facilitates quality control and ultimately approval by the regulatory authorities.

The production of chemically defined peptides can be carried out economically on a large scale. Peptide preparations can be stored freeze-dried, which avoids the need to maintain a 'cold-chain' facility in storage, transport and distribution. There is no risk of reversion or formation of adverse reassortants that can lead to virulence, which is a potential problem associated with live attenuated vaccine preparations.

Peptide-based vaccines can be designed to include multiple determinants from several pathogens, or multiple epitopes from the same pathogen. The introduction of non-natural amino acids and peptide-like molecules into peptide-based vaccines allows the design of more drug-like compounds, which opens up avenues for vaccine delivery and rational drug design in vaccinology.

Despite the numerous advantages associated with the use of peptide vaccines, challenges in peptide vaccination strategies are, for example, the often low immunogenicity of the peptide, especially in the case of tumour antigens, the delivery of peptide epitopes to antigen presenting cells and premature peptide degradation by protease activity in the periphery or in APCs.

Modification of anchor amino acids by other naturally occurring amino acids may result in enhanced binding to the MHC and -together with peptides in which TCR binding is altered- such peptides are designated altered peptide ligands or APLs. Substitutions in the TCR interacting region by naturally occurring amino acid, or heteroclitic analogues, may cause hyperstimulation of the CTL thereby providing a more potent immune response compared with the native epitope. Alternatively heteroclitic analogues may antagonise autoreactive CTLs, leading to immunosuppression, which can be exploited for the treatment of autoimmune disease and prevention of organ rejection following allogeneic transplantation

Another strategy to improve the efficacy of peptide vaccines is the introduction into the peptide of non-naturally occurring amino acid residues, including incorporation of non-encoded alpha amino acids, photoreactive cross-linking of amino acids, beta-amino acids, backbone reduction, partial retro-inversion and incorporation of D-amino acids, N-terminal methylation and C-terminal amidation and pegylation. Synthetic engineering of peptide epitopes thus confers beneficial properties to the peptide vaccine such as improved MHC class I binding and TCR avidity, protease resistance, and oral bioavailability.

Immune restricted peptides, besides in vaccines, can also be used in immunosuppressive therapy and T cell antagonism. Currently, broad-spectrum drugs that generally suppress the immune system are used to reduce the risk of rejection after allogeneic organ transplantation (host versus graft reaction) or to lower the risk of Graft-versus-Host Disease after hematopoietic stem cell (bone marrow) transplantation.

CD8⁺ T cells have been implicated in mediating Graft-versus-Host Disease, but also early allograft rejection, indicating an important role for MHC class I. Also the treatment of autoimmune diseases is based on immunosuppression. The selective knock-down of autoimmune or rejective responses is desirable and hitherto research has been focused on the design of modified versions of the natural pathogenic viral or self-antigenic peptides.

These altered peptide ligands (APLs) are epitopes in which one or multiple of the naturally occurring amino acid residues are replaced by another amino acid residue. They either block the MHC peptide binding groove, inhibiting binding to the TCR, or they antagonise the TCR, i.e. interaction with the TCR does take place, but without the onset of signalling.

Optimisation of immunogenic peptides is valuable for the generation of MHC multimers, which are widely used for epitope restricted T cell detection and isolation for adoptive T cell therapy.

Typically, a substantial proportion of T cell defined tumour-derived antigenic peptides are suboptimal for binding to HLA, with consequent fast dissociation from MHC and weak immunogenicity. Non-natural amino acid substitutions increase peptide binding to MHC resulting in highly stable complexes. It has been observed that the half-life of MHC/peptide complexes is directly correlated to immunogenicity. MHC multimers containing optimised tumour derived antigens, i.e. immunogenic peptides, aid in the isolation and subsequent expansion of, for example, tumour infiltrating lymphocytes.

Considering the above, it is an object of the present invention, amongst other objects, to provide immunogenic peptides with improved efficiency in, for example, vaccines, immunosuppressive therapy, adoptive T cell therapy and diagnostics.

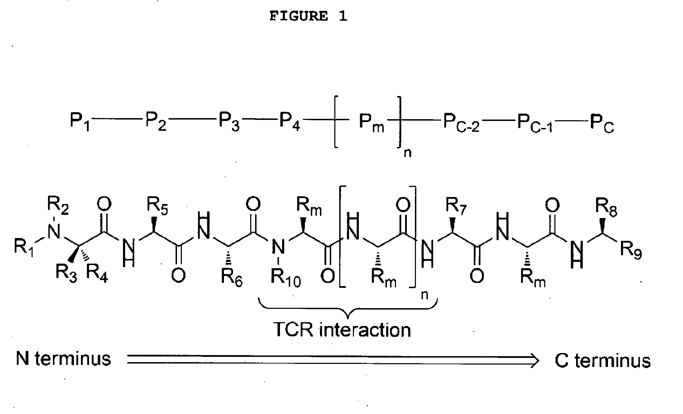

JACS 131(34), 8 May 2009, pages 13205-13213 discloses a series of peptides where use is made of periodate in order to use it as a binder of MHC. Analogue peptides are prepared in JACS 131(34), 8 May 2009, pages 12298-12304 (see Figure 1). Applications, both diagnostic and therapeutic ones, are suggested in the abstract and discussion of D1.

Specifically, the present invention enables a new vaccination technology based on stable peptides that have the ability to induce T cell activation at very low epitope concentrations and/or at late time points after epitope binding to antigen-presenting cells, as an initial prevention against major health threats such as pandemic influenza. In addition, high burden diseases including cancer, such as melanoma, can be targeted with the present peptides.

Further, the present peptides enable inactivation of T cells by blocking the MHC-TCR interaction or by antagonising the T cell receptor.

Furthermore, the present peptides contribute to enhancing MHC multimer technology which is fundamental technique in monitoring infection and cancer, determining vaccination efficiencies and evaluating and isolating T cells for adoptive T cell therapy.

The above objects, amongst other objects, are met by immune restricted peptides as defined in the appended claim 1.

The term "immune restricted peptides", within the context of the present invention, designates modified peptides capable of eliciting, or modifying an immune response. The modification of the present peptides comprises the replacement, or substitution, of one or more amino acids in a peptide, i.e. a peptide representing one or more immunogenic epitopes, with non-naturally occurring amino acids. The present immune restricted peptides can provide an increased immunogenicity as compared the original peptide or are capable to provide immunogenicity to original non-immunogenic peptides.

The term "non-naturally occurring amino acids" within the context of the present invention denotes amino acids which are not found in naturally occurring compounds such as proteins and peptides. Specifically, non-naturally occurring amino acids according to the present invention are not the L-amino acids: alanine, cysteine, aspartic acid, glutamic acid, phenylalanine, glycine, histidine, isoleucine, lysine, leucine, methionine, asparagine, proline, glutamine, arginine, serine, threonine, valine, tryptophan and tyrosine.

Specifically, the above objects, amongst other objects, are met by immune restricted peptides, preferably HLA-A2 restricted peptides, according to the general formula (I) : 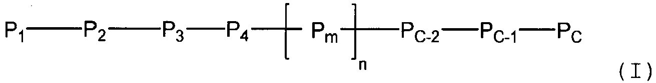 wherein:
- P₁ is selected from the group consisting of am-phg, PHG, 3-PYRA, 3-THI, SOME, CSCF3 and CSME;
- P₂ is a naturally, or non-naturally, occurring amino acid comprising a hydrophobic linear or branched or fluorinated substitution;
- P₃ is a naturally, or non-naturally, occurring amino acid comprising a hydrophobic linear or branched substitution;
- P₄ is a naturally, or non-naturally, occurring amino acid comprising a methylated alpha nitrogen atom;
- Pₘ is a naturally occurring amino acid, n is an integer of 1 to 9, preferably 1 to 4, more preferably 2 or 3;
- P_{C-2} is a naturally, or non-naturally, occurring amino acid comprising a fluorinated aromatic substitution;
- P_{C-1} is a naturally, or non-naturally, occurring amino acid;
- P_{C} is a naturally, or non-naturally, occurring amino acid comprising unsaturated or saturated side chains and/or carboxyl isosteresor a saturated linear carbon chain containing 2 to 4 carbon atoms with or without an oxygen atom or a sulphur atom within the chain and/or a carboxyl isostere.

The present peptides are based on chemically enhanced and/or stabilised variants of immunogenic or non-immunogenic peptides also designated as 'epitopes'. Chemical enhancement and stabilisation of epitopes comprises the incorporation of non-naturally occurring amino acids. The present chemical enhancement and stabilisation of epitopes, or peptides, results, for example, in an improved proteolytic stability and/or enhanced HLA affinity, providing an enhanced immunogenicity and/or T cell antagonism as compared to the original, or non-modified, peptide.

The present invention preferably relates to HLA-A2 restricted epitopes, or HLA-A2 immune restricted peptides, with enhanced affinity for HLA-A2 comprising 8- to 16-, preferably 8- to 13-, more preferably of 9- or 10-mer peptides, based on naturally occurring HLA-A2 restricted epitopes in which at least one amino acid has been replaced by a non-natural modification thereof.

To generate the present immunogenic epitopes, the modifications, as defined above, are introduced on P₁ and can be introduced on amino acids P₂ and/or P₃ (counting from the N-terminus) and on the last (P_{C}) and second before last (P_{C-2}) amino acid. Amino acids between P₃ and the before last amino acid residue (P_{C-2}) are essential for T cell receptor activation.

P_{C-1} generally is any of the standard 20 naturally occurring side chains. Although substitution of this position provides improved binding to the HLA proteins, non-naturally occurring modifications on this position do not lead to activation of T cells. However, non-naturally occurring substitutions at P_{C-1} are beneficial for the development of T cell antagonists. Accordingly, an additional modification of the naturally occurring amino acid at this position by a non-naturally occurring amino acid is contemplated within the context of the present invention.

According to a preferred embodiment, the present invention relates to immune restricted peptides, preferably HLA-A2 Immune restricted peptides, wherein further at least one, preferably at least two, more preferably at least 3, most preferably 4 of P₂, P₃, P₄, P_{C-2} and P_{C} are a non-naturally occurring amino acid.

Specifically preferred combinations of P₁, P₂, P₃, P₄, P_{C-2} and P_{C} are modification of P₁ in combination with P₂ and P_{c}, P₁ in combination with P₂, P_{C-2} and P_{c}, P₂ in combination with P_{C-2} and P_{c}, P₂ in combination with P_{c}, P₂ in combination with P_{C-2}, P₁ in combination with P₂, P₁ in combination with P_{C-2} and P₂ in combination with P_{C-2}.

Preferred examples of non-naturally occurring amino acid combinations at the indicated positions as defined herein are:

| P₁ | P₂ | P_{c} |
|---|---|---|
| [3-PYRA] | [2-AOC] | [PRG] |
| [3-PYRA] | [2-AOC] | |
| [3-PYRA] | [BUTALA] | [PRG] |
| [3-PYRA] | [BUTALA] | |
| [3-PYRA] | [CpALA] | [PRG] |
| [3-PYRA] | [CpALA] | |
| [3-PYRA] | [NLE] | [PRG] |
| [3-PYRA] | [NLE] | |
| [3-PYRA] | [NVA] | [PRG] |
| [3-PYRA] | [NVA] | |
| [3-PYRA] | [3f-ABU] | [PRG] |
| [3-PYRA] | [3f-ABU] | |
| [am-phg] | [2-AOC] | [PRG] |
| [am-phg] | [2-AOC] | |
| [am-phg] | [BUTALA] | [PRG] |
| [am-phg] | [BUTALA] | |
| [am-phg] | [CpALA] | [PRG] |
| [am-phg] | [CpALA] | |
| [am-phg] | [NLE] | [PRG] |
| [am-phg] | [NLE] | |
| [am-phg] | [NVA] | [PRG] |
| [am-phg] | [NVA] | |
| [am-phg] | [3f-ABU] | [PRG] |
| [am-phg] | [3f-ABU] | |
| [CSCF3] | [2-AOC] | [PRG] |
| [CSCF3] | [2-AOC] | |
| [CSCF3] | [BUTALA] | [PRG] |
| [CSCF3] | [BUTALA] | |
| [CSCF3] | [CpALA] | [PRG] |
| [CSCF3] | [CpALA] | |
| [CSCF3] | [NLE] | [PRG] |
| [CSCF3] | [NLE] | |
| [CSCF3] | [NVA] | [PRG] |
| [CSCF3] | [NVA] | |
| [CSCF3] | [3f-ABU] | [PRG] |
| [CSCF3] | [3f-ABU] | |
| [CSME] | [2-AOC] | [PRG] |
| [CSME] | [2-AOC] | |
| [CSME] | [BUTALA] | [PRG] |
| [CSME] | [BUTALA] | |
| [CSME] | [CpALA] | [PRG] |
| [CSME] | [CpALA] | |
| [CSME] | [NLE] | [PRG] |
| [CSME] | [NLE] | |
| [CSME] | [NVA] | [PRG] |
| [CSME] | [NVA] | |
| [CSME] | [3f-ABU] | [PRG] |
| [CSME] | [3f-ABU] | |
| [PHG] | [2-AOC] | [PRG] |
| [PHG] | [2-AOC] | |
| [PHG] | [BUTALA] | [PRG] |
| [PHG] | [BUTALA] | |
| [PHG] | [CpALA] | [PRG] |
| [PHG] | [CpALA] | |
| [PHG] | [NLE] | [PRG] |
| [PHG] | [NLE] | |
| [PHG] | [NVA] | [PRG] |
| [PHG] | [NVA] | |
| [PHG] | [3f-ABU] | [PRG] |
| [PHG] | [3f-ABU] | |
| [SOME] | [2-AOC] | [PRG] |
| [SOME] | [2-AOC] | |
| [SOME] | [BUTALA] | [PRG] |
| [SOME] | [BUTALA] | |
| [SOME] | [CpALA] | [PRG] |
| [SOME] | [CpALA] | |
| [SOME] | [NLE] | [PRG] |
| [SOME] | [NLE] | |
| [SOME] | [NVA] | [PRG] |
| [SOME] | [NVA] | |
| [SOME] | [3f-ABU] | [PRG] |
| [SOME] | [3f-ABU] | |

According to another preferred embodiment of the present invention: 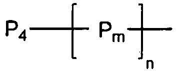 is defined as the residues that make up the bulk part of the interaction site between peptide and TCR, and is preferably part of an HLA-A2 restricted immunogenic epitope. An immunogenic epitope according to the present invention is an amino acid sequence capable of T cell activation. Analogous, an HLA-A2 immunogenic epitope according to the present invention is an amino acid sequence capable of T cell activation through HLA-A2 presentation.

According to yet another preferred embodiment, the present invention relates to immune restricted peptides, preferably HLA-A2 immune restricted peptides, according to the general formula (II): 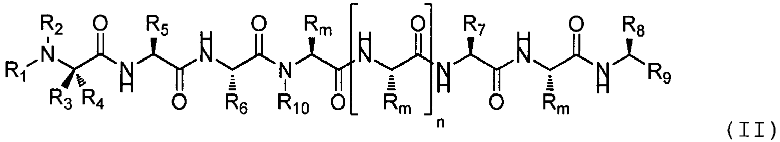 wherein:
- R₁ to R₄ are as defined by am-phg, PHG, 3-PYRA, 3-THI, SOME, CSCF3 or CSME;
- R₅ is a fluorinated or non-fluorinated saturated linear aliphatic chain containing 2 to 6 carbon atoms with or without an oxygen atom or a sulfur atom within the chain or is defined as in 3F-ABU;
- R₆ is a saturated linear aliphatic chain containing 2 to 6 carbon atoms; and/or
- R₇ is a fluorinated benzyl moiety; and/or
- R₈ is an unsaturated carbon chain comprising of 2 to 3 carbon atoms and R₉ is carboxyl; and/or
- R₈ is a saturated or unsaturated linear or branched carbon chain containing 2 to 4 carbon atoms with or without an oxygen atom or a sulphur atom within the chain, or a terminal thiol group and R₉ is selected from the group consisting of carboxylate, tetrazole, boronate, phosphonate, N-acyl-S-alkyl-sulfonamides (specifically N-acyl-S-methyl-sulfonamide), and hydroxamate; and/or
- R₁₀ is H or methyl; and/or
- Rₘ is a naturally occurring amino acid side chain.

According to the present invention, the present non-naturally occurring amino acid according to the present invention are preferably selected from the group consisting of TIC, CSME, OM-HS, NVA, NLE, BUTALA, PRG, PHG, SOME, 2-AOC, CₚALA, ALG, am-phg, 3-PYRA, 3-THI, 3F-ABU, CSCF3 and 4-FPHE.

According to especially preferred embodiments of the present invention: P_{C-2} is 4-FPHE, P₂ is selected from the group consisting of CₚALA, NLE, BUTALA, NVA, 3F-ABU, (L)3F-ABU and 2-AOC, P₃ is NLE, P₄ is an alpha-N-methylated amino acid residue containing a naturally occurring side-chain and/or P_{C} is selected from the group consisting of ALG, PRG, NLE, CSME and OM-HS and any combination of the indicated non-naturally occurring amino acids at the position P_{C-2}, P₂, P₃, P₄ and P_{c}.

The preferred immunogenic epitopes according to the present invention as represented by 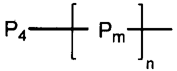 are GFV, part of the HLA-A2 restricted influenza A matrix protein 1 (58-66) epitope, GIGI, part of the HLA-A2 restricted melanoma Mart-1 (26-35)epitope, or DFF, part of the HLA-A2 restricted melanoma TRP-2 (180-188) HLA-A2 epitope.

Considering the beneficial properties of the present immunogenic restricted, or modified, peptides according to the present invention, especially in the fields of vaccines, immunosuppressive therapy, adoptive T cell therapy and/or diagnostics, the present invention, according to another aspect, relates to a method for providing a immune restricted peptide, preferably an HLA-A2 restricted immunogenic peptide, comprising:
a) selecting an immunogenic peptide, preferably an HLA-A2 immunogenic peptide, represented by the formula 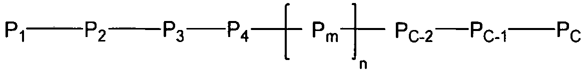 wherein P₁, P₂, P₃, P₄, Pₘ, P_{C-2} P_{C-1} and P_{C} are naturally occurring amino acids;
b) replacing at least one of the naturally occurring amino acids at position P₁ and, preferably, at least one of the positions P₂, P₃, P₄, P_{C-2} and P_{C} by a non-naturally occurring amino acid as defined in any of the claims 1 to 10 thereby providing an immune restricted peptide, preferably an HLA-A2 restricted peptide
wherein the replacement of P₁ is selected from the group consisting of am-phg, PHG, 3-PYRA, 3-THI, SOME, CSCF3 and CSME.

According to a preferred embodiment of the present method, the method comprises further comprising after step (a), but before step (b), analysing the amino acid sequence of the immunogenic peptide using a computer algorithm providing, preferably, a prediction of the at least one of the naturally occurring amino acids at positions P₂, P₃, P₄, P_{C-2} and P_{C} to be replaced by the non-naturally occurring amino acid and the identification of the replacement non-naturally occurring amino acid at positions P₁, P₂, P₃, P₄, P_{C-2} and/or P_{C}.

According to another preferred embodiment of this aspect, the present invention relates to a method wherein step (b) comprises replacing at least two, preferably at least three, more preferably at least four naturally occurring amino acids at positions P₂, P₃, P₄, P_{C-2} and P_{C}.

Specifically preferred combinations of P₁, P₂, P₃, P_{C-2} and P_{C} are modification of P₁ in combination with P₂ and P_{C}, P₁ in combination with P₂, P_{C-2} and P_{C}, P₂ in combination with P_{C-2} and P_{c}, P₂ in combination with P_{c}, P₂ in combination with P_{C-2}, P₁ in combination with P₂, P₁ in combination with P_{C-2} and P₂ in combination with P_{C-2}.

Preferred examples of non-naturally occurring amino acid combinations at the indicated positions as defined herein are:

| P₁ | P₂ | P_{c} |
|---|---|---|
| [3-PYRA] | [2-AOC] | [PRG] |
| [3-PYRA] | [2-AOC] | |
| [3-PYRA] | [BUTALA] | [PRG] |
| [3-PYRA] | [BUTALA] | |
| [3-PYRA] | [CpALA] | [PRG] |
| [3-PYRA] | [CpALA] | |
| [3-PYRA] | [NLE] | [PRG] |
| [3-PYRA] | [NLE] | |
| [3-PYRA] | [NVA] | [PRG] |
| [3-PYRA] | [NVA] | |
| [3-PYRA] | [3f-ABU] | [PRG] |
| [3-PYRA] | [3f-ABU] | |
| [am-phg] | [2-AOC] | [PRG] |
| [am-phg] | [2-AOC] | |
| [am-phg] | [BUTALA] | [PRG] |
| [am-phg] | [BUTALA] | |
| [am-phg] | [CpALA] | [PRG] |
| [am-phg] | [CpALA] | |
| [am-phg] | [NLE] | [PRG] |
| [am-phg] | [NLE] | |
| [am-phg] | [NVA] | [PRG] |
| [am-phg] | [NVA] | |
| [am-phg] | [3f-ABU] | [PRG] |
| [am-phg] | [3f-ABU] | |
| [CSCF3] | [2-AOC] | [PRG] |
| [CSCF3] | [2-AOC] | |
| [CSCF3] | [BUTALA] | [PRG] |
| [CSCF3] | [BUTALA] | |
| [CSCF3] | [CpALA] | [PRG] |
| [CSCF3] | [CpALA] | |
| [CSCF3] | [NLE] | [PRG] |
| [CSCF3] | [NLE] | |
| [CSCF3] | [NVA] | [PRG] |
| [CSCF3] | [NVA] | |
| [CSCF3] | [3f-ABU] | [PRG] |
| [CSCF3] | [3f-ABU] | |
| [CSME] | [2-AOC] | [PRG] |
| [CSME] | [2-AOC] | |
| [CSME] | [BUTALA] | [PRG] |
| [CSME] | [BUTALA] | |
| [CSME] | [CpALA] | [PRG] |
| [CSME] | [CpALA] | |
| [CSME] | [NLE] | [PRG] |
| [CSME] | [NLE] | |
| [CSME] | [NVA] | [PRG] |
| [CSME] | [NVA] | |
| [CSME] | [3f-ABU] | [PRG] |
| [CSME] | [3f-ABU] | |
| [PHG] | [2-AOC] | [PRG] |
| [PHG] | [2-AOC] | |
| [PHG] | [BUTALA] | [PRG] |
| [PHG] | [BUTALA] | |
| [PHG] | [CpALA] | [PRG] |
| [PHG] | [CpALA] | |
| [PHG] | [NLE] | [PRG] |
| [PHG] | [NLE] | |
| [PHG] | [NVA] | [PRG] |
| [PHG] | [NVA] | |
| [PHG] | [3f-ABU] | [PRG] |
| [PHG] | [3f-ABU] | |
| [SOME] | [2-AOC] | [PRG] |
| [SOME] | [2-AOC] | |
| [SOME] | [BUTALA] | [PRG] |
| [SOME] | [BUTALA] | |
| [SOME] | [CpALA] | [PRG] |
| [SOME] | [CpALA] | |
| [SOME] | [NLE] | [PRG] |
| [SOME] | [NLE] | |
| [SOME] | [NVA] | [PRG] |
| [SOME] | [NVA] | |
| [SOME] | [3f-ABU] | [PRG] |
| [SOME] | [3f-ABU] | |

According to the present invention, the present non-naturally occurring amino acid substitutions at positions P₁, P₂, P₃, P_{C-2} and P_{C} are preferably selected from the group consisting of TIC, CSME, OM-HS, NVA, NLE, BUTALA, PRG, PHG, SOME, 2-AOC, CₚALA, ALG, am-phg, 3-PYRA, 3-THI, 3F-ABU, CSCF3 and 4-FPHE.

According to an especially preferred embodiments of the present invention the replacement non-naturally occurring amino acid at position P_{C-2} is 4-FPHE, the replacement non-naturally occurring amino acid at position P₂ is selected from the group consisting of CₚALA, NLE, BUTALA, NVA, 3F-ABU, (L)3F-ABU, CSME and 2-AOC, the replacement non-naturally occurring amino acid at position P₃ is NLE, the replacement non-naturally occurring amino acid at position P₄ is an alpha N-methylated amino acid containing a naturally occurring side chain and/or the replacement non-naturally occurring amino acid at position P_{C} is selected from the group consisting of ALG, PRG, NLE, and OM-HS or any combination of the indicated replacement non-naturally occurring amino acid at their respective positions.

The present variant or modified peptides provide beneficial properties especially in the fields of vaccines, immunosuppressive therapy, adoptive T cell therapy and diagnostics. Accordingly, according to another aspect, the present invention relates to the use of the present immune restricted peptides in medicine.

Preferably, the present immune restricted peptides are in vaccines, in immunosuppressive therapy or T cell antagonism, diagnostic and/or in adoptive T cell therapy.

The present invention will be further detailed in the examples below outlining especially preferred embodiments of the present immune restricted peptides. In the examples, reference is made to figures wherein:
- **Figure 1:**: is a schematic representation of HLA binding peptides comprising non-naturally occurring amino acid modifications enhancing HLA binding affinity;
- **Figure 2:**: shows a representative example of a flow cytometry output image. T cells can be CD8 negative (lower left quadrant), CD8 positive (APC) but not MHC tetramer positive (streptavidin-PE), CD8 positive (FITC), but not interferon-γ (APC) positive (upper left quadrant) or double positive (upper right quadrant);
- **Figure 3:**: shows the chemical structures of preferred non-naturally occurring amino acids, their IUPAC names and their abbreviations;
- **Figure 4:**: shows a schematic representation of a T cell activation time course assay. Interferon-γ production was determined at several time points;

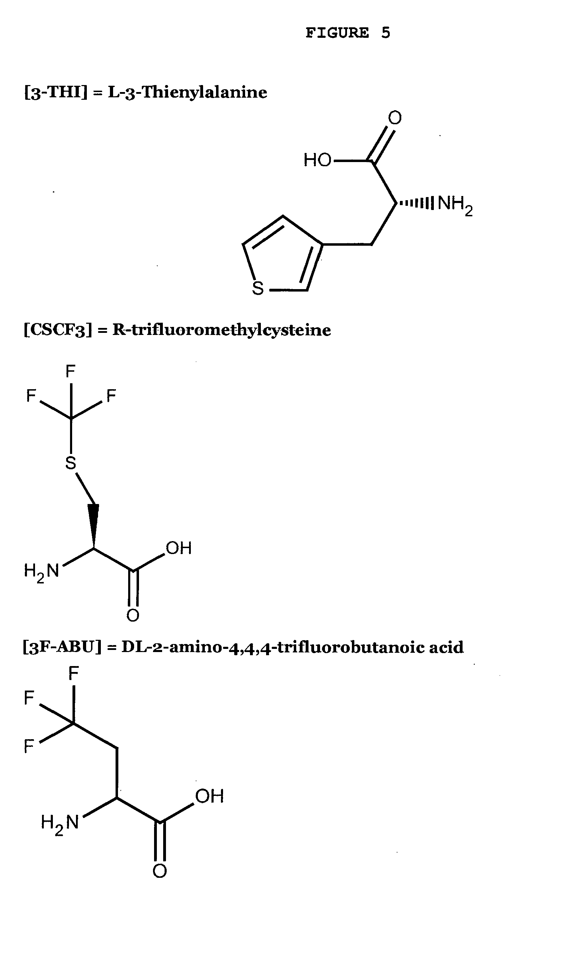

- **Figure 5:**: shows the chemical structures of preferred non-naturally occurring amino acids 3-THI, CSCF3 and 3F-ABU;
- **Figure 6:**: shows the results of a vaccination of mice with WT peptide ELAGIGILTV or modified peptide [am-phg][NVA]AGIGILT[PRG];
- **Figure 7:**: shows the results of a vaccination of mice with WT peptide LLFGLALIEV or modified peptide [PHG][2-AOC]FGLALIEV.
- **Figure 8:**: shows the results of a vaccination of mice with WT peptide ALKDVEERV or modified peptides [PHG][2-AOC]KDVEERV or [CSME][2-AOC]KDVEERV;

### EXAMPLES

### Introduction

In the examples below, optimisation of HLA A2 epitopes (**Figure 1**) is evaluated using the following techniques: HLA binding affinity of peptides is determined using an MHC exchange fluorescence polarisation assay. Binding of peptide MHC to T cells is assessed using MHC multimer technology. T cell activation by chemically enhanced epitopes is determined using an interferon-γ (IFNγ) production assay. IFNγ is a cytokine, predominantly produced upon T cell activation.

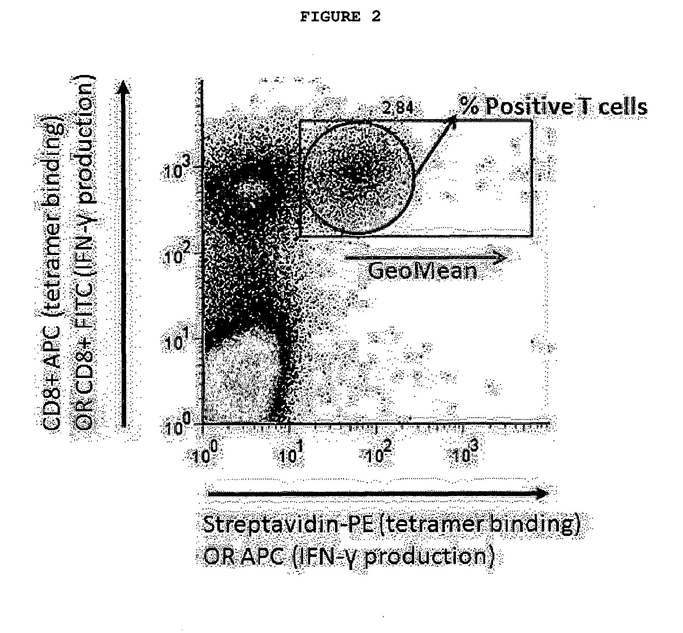

Both the IFNγ and MHC tetramer assays are monitored using flow cytometry, in which fluorescently labelled cells can be detected (**Figure 2**)**.** MHC-TCR interaction is visualised by phycoerythrin (PE) conjugated MHC-streptavidin tetramers and allophycocyanin (APC) conjugated anti-CD8 antibody capable of staining CD8+ T cells. Double positive cells are indicative of CD8+ T cells bound to peptide-MHC tetramers. Both the percentage of tetramer binding CD8+ T cells and the efficiency of tetramer staining per T cell, represented by the geometric mean (displayed as arbitrary fluorescence units) are taken into account.

IFNγ production is visualised by intracellular staining using an APC conjugated anti-IFNγ antibody, whereas the CD8+ T cell is stained with a fluorescein isothiocyanate (FITC)labelled anti-CD8 antibody. Both the percentage of IFNγ producing T cells and the amount of IFNγ produced per T cell (represented by the geometric mean) are taken into account.

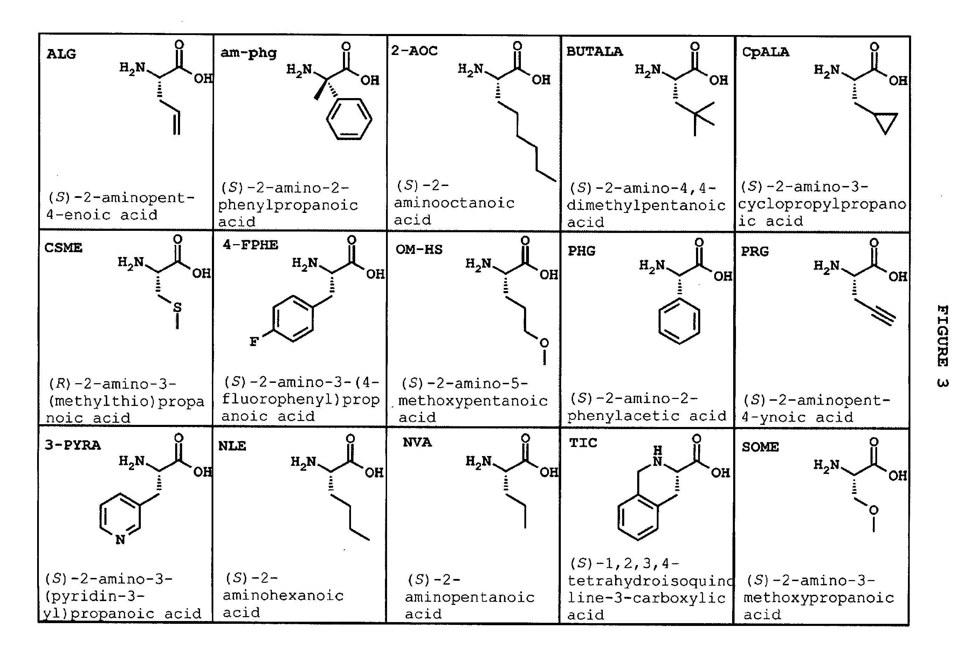

In the examples below, T cell receptor exposed residues are left unchanged in order to maintain immunogenicity. The immunogenic activity of both high and low affinity epitopes has been enhanced with relative ease. An increase in HLA binding affinity up to a factor 1000 has been achieved. Epitopes enhanced by the invented technology presented here showed increased T cell stimulatory activity, as determined by IFNγ production, compared to native epitopes. The chemical structures of the non-naturally occurring amino acids used below are presented in **Figure 3****.**

### Material and methods

HLA binding affinity was determined by a fluorescence polarization (FP) assay based on UV mediated MHC peptide exchange. In short, purified soluble MHC class I molecules (HLA-A0201) loaded with a UV-labile peptide KILGFVFJV, in which J is photocleavable 3-amino-3-(2-nitrophenyl)propionic acid, (5.3 µM stock) are used for this assay. MHC molecules are diluted in phosphate buffer saline containing 0.5 mg/ml bovine gamma globulines (referred to as PBS/BGG) to a final concentration of 0.75 µM and pipetted into a 96 well microplate.

The HLA-A2 restricted hepatitis B virus epitope, FLPSDCFPSV, fluorescently labelled with tetramethylrhodamine (TAMRA) covalently bound to the cysteine residue, is used as the tracer. This tracer peptide is diluted in PBS/BGG to a concentration of 6 nM and manually pipetted into a 96 well microplate. The peptides of interest are diluted in DMSO to a concentration of 125 µM and pipetted into a 96 well microplate. A Hamilton high throughput liquid handling robot is then used combine the components from the three 96 well microplates into a black nonbinding surface 384 well microplate so that each peptide can be measured in triplicate for the fluorescence polarization assay. Once all the components are in the 384 well microplate (30 µl per well of 0.5 µM MHC, 1 nM tracer and 5 µM peptide), the plate is spun down to mix all the components and to remove any air bubbles.

Competition between the tracer peptide and the peptides of interest starts when the 384 well microplate is placed under a UV-lamp (> 350 nm) for 30 minutes at 4 °C to cleave the UV-labile peptide.

All scores represent the percentage inhibition of the FP of the fluorescent tracer peptide. IC₅₀ values are represented as fold increase towards the index peptide, which is set to an arbitrary value of 1.

Peptide/MHC (p/MHC) binding to the TCR was analysed by Fluorescence Assisted Cell Sorting (FACS) on a BD FACSCalibur machine, where 20,000 to 30,000 events were counted per sample. In short, enhanced and control peptides were pipetted in DMSO to a final concentration of 500 µM in a 96 well microplate. Biotinylated MHC monomers (2.45 mg/ml stock) were then diluted in PBS to 25 µg/ml and dispensed with non-binding surface pipette tips, 27 µl/per well in a 96 well microplate. 3 µl of the peptide plate was added to the MHC monomer plate and UV-irradiated for 30 minutes. The plates were then left at RT for another 30 minutes.

Subsequently, the plates were centrifuged for 5 minutes at 3300 RCF to remove disintegrated MHC molecules and 20 µl supernatant was transferred to a new 96 well microplate. 20 µl of PBS-diluted streptavidin-R-phycoerythrin conjugate (27 µg/ml) was added to the peptide-MHC plate in 4 x 15 minute intervals. The intervals are necessary to saturate the streptavidin molecules with the biotinylated MHC molecules so that the maximum amount of fully loaded tetramers is achieved.

In the same time, 100,000 T cells per well were plated out by a Thermo Scientific wellmate cell dispenser in a 384 well microplate. A Hamilton high throughput liquid handling robot was used to add 2 µl of p/MHC-tetramer in triplicate from the 96 well microplate into the cell-filled 384 well microplate. This plate was then incubated for 15 minutes at 37°C. Then 4 µl of allophycocyanin conjugated anti-CD8 antibody (8 x diluted in PBS) was added to each well and incubated for 20 minutes on ice. Subsequently, after two wash steps with PBS, the wells were filled with PBS containing PI to distinguish between live and dead T cells in the FACS analysis. Data were analysed using FCS Express 2 by De Novo software and Microsoft Excel.

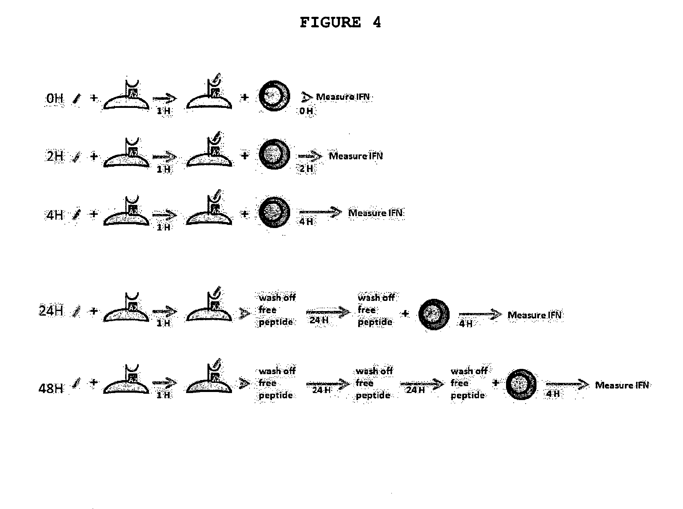

T cell activation assays (**Figure 4**) based on IFNγ production were carried out using a BD Cytofix/Cytoperm™ Fixation/Permeabilization Solution Kit with BD GolgiPlug™. FACS was employed to obtain results. As antigen presenting cells, T2 cells or JY cells were used, pulsed with different concentrations of our peptides for the duration of one hour at 37°C.

T2 cells were used as antigen presenting platform and were cultured in RPMI medium containing 10% fetal bovine serum supplemented with penicillin and streptomycin. T cells were grown in RPMI/AIM-V medium (50:50), supplemented with 10% human serum, penicillin and streptomycin, interleukin-2 and glutamax. 50,000 T2 cells were plated out per well and peptides were added to a 1 µm final concentration. T2 cells and peptides were incubated at 37 °C for 1H after which 50,000 T cells in 50 µl medium were added to the T2 plate. 100 µl of RPMI medium containing 10% fetal bovine serum supplemented with penicillin and streptomycin was added to the wells and the plate was spun at 1500 rpm for 3 minutes. The supernatant was discarded and 100 µl of GolgiPlug (1 µl/ml) in RPMI medium containing 10% fetal bovine serum supplemented with penicillin and streptomycin was added to the cells. For the positive control, 2 µl of phorbol 12-myristate 13-acetate (PMA) and 2 µl of ionomycin diluted in GolgiPlug medium was added to the positive control cells. The plate was spun at 700 rpm for 2 minutes and incubated for 4 hours at 37 °C.

After incubation the plate was spun at 1300 rpm for 3 minutes and the supernatant was discarded. The cells were resuspended in 50 µl of FACS buffer with FITC labelled anti-CD8 antibody (20 µl/ml) and left to stain for 15 minutes in the dark at room temperature.

After staining the plate was spun at 1300 rpm for 3 minutes, and two wash steps were performed in which the cells are washed with 300 µl of FACS buffer. The cells were resuspended in 100 µl of Cytofix/Cytoperm solution and incubated on ice for 20 minutes. The plate was spun at 1300 rpm for 3 minutes and the supernatant was discarded and replaced by 250 µl of Permwash; this step was repeated. The cells were resuspended in 50 µl of Permwash with APC conjugated anti-IFNγ antibody. PermWash buffer was used for the dilution of the APC conjugated anti-IFNγ antibody, rather than a standard buffer, in order to maintain cells in a permeabilised state for the intracellular staining. The plate was incubated on ice for 30 minutes. The plate was spun at 1300 rpm for 3 minutes and the supernatant was discarded and replaced by 250 µl of Permwash; this step was repeated.

After the final wash step, the supernatant was discarded and the cells were resuspended in FACS buffer. Cells were then transferred from the plate into FACS-tubes and the samples were analysed by FACS. Data were analysed using FCS Express 2 by De Novo software and Microsoft Excel.

T cell activation results from table 7 were determined by measuring Interferon-γ using an Enzyme-linked immunosorbant assay (ELISA) as described in Kuiper et al., Am J Ophthalmol, 2011. Biotinylated Interferon-γ antibodies were added to activated T cells, these were incubated with streptavidin R-phycoerythrin after which fluorescence was analyzed.

### Example 1: GILGFVFTL - Influenza A, Matrix Protein 1, residues 58-66

The Influenza A Matrix 1 epitope is a highly conserved epitope amongst Influenza A variants and binds strongly to HLA-A2.1. This epitope serves as a model for stringent selection of unnatural amino acid modifications. Modifications and evaluation of HLA binding and T cell reactivity are summarised in **Table 1.** Replacements found to enhance the HLA affinity of this epitope, were also found to be beneficial to HLA binding of other epitopes (see examples 2 and 3 below).

**Table 1: HLA affinity and T cell recognition of, and T cell activation by optimized Influenza A, Matrix Protein 1 (58-66) analogues.**

| Entry | Sequence | % inhibitio n at T=4H and T=24H | | IC₅₀ Ratio | % Positive TCR recognition | Geometric mean IFN in arbitrary fluorescence units | % CD8+ T cells producing IFN-γ | Geometric mean IFN-γ in arbitrary fluorescence units |
|---|---|---|---|---|---|---|---|---|
| | | F P 4 h | FP 24h | | % TCR | Geo TCR | % IFN | Geo IFN |
| 1 | [am-phg]ILGFV[4-FPHE]TL | 92 | 93 | 6 | 3.27 | 197 | 2.2 | 208 |
| 2 | [am-phg][CpALA]LGFV[4-FPHE]TL | 96 | 96 | 5 | 3.26 | 208 | 2.8 | 243 |
| 3 | [PHG][NLE]LGFVFTL | 77 | 77 | 4 | 3.58 | 328 | 2.7 | 238 |
| 4 | [am-phg][BUTALA]LGFV[4-FPHE]TL | 83 | 84 | 3 | 3.20 | 217 | 2.6 | 266 |
| 5 | [PHG]I[NLE]GFVFTL | 86 | 84 | 3 | 3.26 | 235 | 2.9 | 270 |
| 6 | [am-phg][NLE]LGFV[4-FPHE]TL | 82 | 83 | 3 | 3.40 | 302 | 3.0 | 221 |
| 7 | [am-phg][BUTALA]LGFVFTL | 9 5 | 96 | 3 | 3.32 | 155 | 2.8 | 205 |
| 8 | [am-phg][NVA]LGFV[4-FPHE]TL | 9 6 | 97 | 2 | 3.54 | 143 | 3.4 | 242 |
| 9 | [PHG][BUTALA]LGFVFTL | 7 4 | 75 | 2 | 3.16 | 464 | 2.5 | 249 |
| 10 | [am-phg][CpALA]LGFVFTL | 9 0 | 91 | 2 | 3.17 | 150 | 2.2 | 259 |
| 11 | GILGFVFTL | 8 1 | 78 | 1 | 3.80 | 219 | 2.6 | 237 |
| 12 | FLPSDFFPSV | 8 9 | 87 | 3 | 0.10 | 100 | 0.0 | 106 |

The columns listed under 'Sequence' numbering 1 to 9 indicate the amino acid residue present on the designated position in the epitope.

CD8⁺ T cells were obtained from Influenza A positive donors and were sorted using tetramers containing HLA A2.1::GILGFVFTL. The hepatitis B viral epitope FLPSDFFPSV (entry 12) was used as a negative control peptide in the TCR binding and IFNγ production assays. This natural epitope is known for its very high affinity for HLA-A2.1.

FP 4H and 24H represent percentage inhibition of tracer peptide binding by 5 µM competitor peptide at 4 hours and 24 hours incubation, respectively. High inhibition values maintained over 24 hours indicate a low off-rate of the peptide and consequently long lived p/MHC complexes.

IC₅₀ values were determined using the MHC exchange FP assay and IC₅₀ ratios represent IC₅₀ values determined using the FP MHC exchange assay normalised to the native index peptide (entry 11).

%TCR shows the percentage of CD8+ T cells that are stained by the indicated p/MHC-tetramers. GeoTCR represents T cell staining efficiency.

The last two columns represent IFNγ production by stimulated T cells. %IFN indicates the percentage of T cells that are both CD8+ and produce IFNγ, whereas GeoIFN indicates the amount of IFNγ per T cell. 1.5 µg peptide was added per well to load antigen presenting cells and IFNγ production at time point 4H after adding T cells to the antigen presenting cells was measured.

In short, all substitutions introduced in Influenza A matrix 1 epitope (58-66) lead to enhanced HLA-A2 affinity, and similar or improved T cell activation efficiency (entries 2 to 10) compared to the native epitope.

### Example 2: EAAGIGILTV - Melanoma, Mart-1, residues 26-35

The melanoma epitope EAAGIGILTV has low HLA affinity. When it became clear that defined anchor residues exist for all specific HLA types, replacement of alanine on P2 by a leucine was used to create an altered peptide ligand with greater MHC affinity, while maintaining T cell activation of lymphocytes that respond to native epitope EAAGIGILTV. The A to L mutation enhances MHC affinity, but not to the extent that is shown below by introduction of unnatural substitutions. Modifications and evaluation of HLA binding and T cell reactivity are summarised in **Table 2.**

**Table 2: HLA affinity, T cell recognition and T cell activation by optimized Melanoma, Mart-1 (26-35) analogues**

| Entry | Sequence | % inhibition at T=4H and T=24H | | | % Positive TCR recognition | Geometric mean in arbitrary fluorescence units | % CD8+ T cells producing IFN peptide concentrations in picomolar | | | | | | Geometric mean IFN in arbitrary fluorescence units peptide concentrations in picomolar | | | | | |
|---|---|---|---|---|---|---|---|---|---|---|---|---|---|---|---|---|---|---|
| | | FP 4h | FP 24h | IC₅₀ Ratio | % TCR | Geo TCR | 100 | 50 | 5 | 0.5 | 0.05 | 0.005 | 100 | 50 | 5 | 0.5 | 0.05 | 0.005 |
| 1 | [CSME][2-AOC]AGIGILTV | 72 | 71 | 12.2 | 11 | 212 | 8 | 8 | 8 | 6 | 2 | 1 | 177 | 179 | 130 | 101 | 76 | 73 |
| 2 | [am-phg][NVA]AGIGILT[ALG] | 58 | 63 | 2.5 | 9 | 321 | 9 | 8 | 6 | 3 | 1 | 1 | 106 | 106 | 82 | 66 | 63 | 72 |
| 3 | [am-phg][NVA]AGIGILT[PRG] | 67 | 64 | 8.3 | 11 | 314 | 6 | 6 | 5 | 3 | 2 | 1 | 139 | 152 | 109 | 82 | 72 | 87 |
| 4 | [am-phg]LAGIGILT[PRG] | 68 | 65 | 7.2 | 10 | 334 | 8 | 6 | 5 | 2 | 1 | 1 | 93 | 94 | 74 | 62 | 70 | 63 |
| 5 | [am-phg][2-AOC]AGIGILT[PRG] | 80 | 77 | 24.3 | 10 | 283 | 5 | 5 | 5 | 2 | 1 | 1 | 162 | 161 | 123 | 82 | 83 | 76 |
| 6 | [4-FPHE]AAGIGI[4-FPHE]TV | 65 | 61 | 1.7 | ND | ND | ND | ND | ND | ND | ND | ND | ND | ND | ND | ND | ND | ND |
| 7 | [am-phg][NVA]AGIGI[4-FPHE]TV | 65 | 63 | 10.1 | 9 | 173 | ND | ND | ND | ND | ND | ND | ND | ND | ND | ND | ND | ND |
| 8 | [am-phg][NLE]AGIGILT[PRG] | 73 | 72 | 6.6 | 11 | 301 | ND | ND | ND | ND | ND | ND | ND | ND | ND | ND | ND | ND |
| 9 | [am-phg]AAGIGI[4-FPHE]TV | 62 | 59 | 2.1 | ND | ND | ND | ND | ND | ND | ND | ND | ND | ND | ND | ND | ND | ND |
| 10 | [CSME][2-AOC]AGIGILT[PRG] | 72 | 70 | 14.8 | 10 | 264 | ND | ND | ND | ND | ND | ND | ND | ND | ND | ND | ND | ND |
| 11 | [CSME][NLE]AGIGILTV | 62 | 60 | 3.2 | 11 | 223 | ND | ND | ND | ND | ND | ND | ND | ND | ND | ND | ND | ND |
| 12 | [CSME][NVA]AGIGILTV | 82 | 77 | 3.2 | 11 | 224 | ND | ND | ND | ND | ND | ND | ND | ND | ND | ND | ND | ND |
| 13 | [CSME][NVA]AGIGILT[PRG] | 63 | 60 | 2.8 | 11 | 200 | ND | ND | ND | ND | ND | ND | ND | ND | ND | ND | ND | ND |
| 14 | [PHG][NVA]AGIGILT[ALG] | 65 | 64 | 18.9 | 9 | 194 | ND | ND | ND | ND | ND | ND | ND | ND | ND | ND | ND | ND |
| 15 | [PHG]AAGIGI[4-FPHE]T[NLE] | 63 | 66 | 6.8 | ND | ND | ND | ND | ND | ND | ND | ND | ND | ND | ND | ND | ND | ND |
| 16 | [PHG]AAGIGI[4-FPHE]TV | 61 | 61 | 1.9 | ND | ND | ND | ND | ND | ND | ND | ND | ND | ND | ND | ND | ND | ND |
| 17 | [SOME][2-AOC]AGIGILTV | 68 | 70 | 10.1 | 10 | 238 | ND | ND | ND | ND | ND | ND | ND | ND | ND | ND | ND | ND |
| 18 | E[NLE]AGIGI[4-FPHE]TV | 63 | 64 | 7.5 | 8 | 217 | ND | ND | ND | ND | ND | ND | ND | ND | ND | ND | ND | ND |
| 19 | E[2-AOC]AGIGI[4-FPHE]TV | 64 | 62 | 5.1 | ND | ND | ND | ND | ND | ND | ND | ND | ND | ND | ND | ND | ND | ND |
| 20 | E[2-AOC]AGIGI[4FPHE]T[OM-HS] | 78 | 66 | 1.9 | 8 | 180 | ND | ND | ND | ND | ND | ND | ND | ND | ND | ND | ND | ND |
| 21 | E[NVA]AGIGI[4-FPHE]TV | 59 | 59 | 1.5 | 9 | 230 | ND | ND | ND | ND | ND | ND | ND | ND | ND | ND | ND | ND |
| 22 | [CSME]LAGIGILTV | 55 | 55 | ND | 12 | 219 | ND | ND | ND | ND | ND | ND | ND | ND | ND | ND | ND | ND |
| 23 | [CSME]LAGIGILT[PRG] | 63 | 58 | ND | 10 | 221 | ND | ND | ND | ND | ND | ND | ND | ND | ND | ND | ND | ND |
| 24 | [CSME][NVA]AGIGILTV | 57 | 56 | ND | 10 | 252 | ND | ND | ND | ND | ND | ND | ND | ND | ND | ND | ND | ND |
| 25 | [CSME]AAGIGI[4-FPHE]TV | 63 | 58 | ND | 8 | 203 | ND | ND | ND | ND | ND | ND | ND | ND | ND | ND | ND | ND |
| 26 | [SOME][NLE]AGIGILTV | 58 | 58 | ND | 10 | 211 | ND | ND | ND | ND | ND | ND | ND | ND | ND | ND | ND | ND |
| 27 | [SOME]LAGIGILTV | 58 | 54 | ND | 9 | 300 | ND | ND | ND | ND | ND | ND | ND | ND | ND | ND | ND | ND |
| 28 | ELAGIGI[4-FPHE]TV | 61 | 57 | ND | 10 | 263 | ND | ND | ND | ND | ND | ND | ND | ND | ND | ND | ND | ND |
| 29 | ELAGIGILTV | 62 | 58 | 1 | 11 | 313 | 9 | 9 | 5 | 2 | 1 | 1 | 108 | 109 | 73 | 64 | 63 | 71 |
| 30 | EAAGIGILTV | 51 | 18 | 0.007 | 11 | 223 | ND | ND | ND | ND | ND | ND | ND | ND | ND | ND | ND | ND |

In the columns listed under 'Sequence' numbering 1 to 10 indicates the amino acid present on the designated position in the epitope. HLA-A2::Mart-1(26-35) reactive T cells were obtained either by transduction of CD8+ T cells with a viral vector containing a monoclonal TCR for EAAGIGILTV or were isolated from melanoma patients and sorted using MHC tetramers containing HLA A2.1::ELAGIGLTV. FP 4H and 24H represent percentage inhibition of tracer peptide binding by 5 µM competitor peptide at 4 hours and 24 hours incubation, respectively. High inhibition values maintained over 24 hours indicate a low off rate of the peptide and consequently long lived p/MHC complexes.

IC₅₀ values were determined using the MHC exchange FP assay and were normalised to the well known A2L altered peptide ligand (ELAGIGILTV), represented as IC₅₀ ratios.

%TCR and GeoTCR are to be interpreted as in **Table 1.** The data shown were obtained using T cells transduced with a viral vector containing a monoclonal TCR for EAAGIGILTV.

%IFN and GeoIFN are to be interpreted as in **Table 1.** Peptide concentrations ranged from 100 pM to 0.005 pM.

The data shown were obtained using FACS-sorted T cells derived from a single patient, thus containing EAAGIGILTV reactive TCRs. The wild type epitope EAAGIGILTV was not included as a control because previous experiments indicated that at the concentrations used in this assay, this peptide did not induce measurable IFNγ expression.

The introduction of multiple nonnatural amino acid residues yielded peptides with up to a factor 24 higher HLA-A2 binding affinity (entry 5) according to the IC₅₀ data compared to the reported A2L modification. Compared to the original wild type epitope a 300-400 fold increase in HLA affinity is observed.

TCR binding data show that most of the optimised peptides display similar or enhanced T cell staining efficiency as compared to native or A2L modified epitopes. It is also observed that [4-FPHE] on P_{C-2} increases HLA affinity but not T cell activation. Whereas in the Influenza epitope [4-FPHE] replaces a phenylalanine which it closely resembles, here it replaces a leucine on a site exposed to the TCR. Apparently, interaction between MHC loaded with this peptide analogue and the TCR is hampered. Consequently, the introduction of [4-FPHE] on P_{C-2} does not constitute a general improvement of immunogenicity, but is dependent on the particular epitope-TCR combination.

Titration of peptide analogues in the IFNγ assay revealed a tenfold higher T cell stimulatory activity of [CSME][2-AOC]AGIGILTV (entry 1) compared to control epitope ELAGIGILTV (entry 29).

After determination of optimal peptide concentrations in the titration assay, an experiment was set up in which interferon-γ production by T cells upon stimulation by APC's (here T2 cells) displaying modified epitopes was monitored over time as is schematically shown in **Figure 4****.** The standard 4 hour timepoint was also taken along. For the 24h and 48h time points free peptide was washed away at the times indicated in **Figure 4** to assess how long MHC complexes presenting the modified epitopes are present at the cell surface of the APC's at a concentration sufficient to induce T cell activation.

The experiment involved the same modified peptides as used earlier in the titration assay and was carried out using peptide concentrations of 50 pM (Table 3)and 0.5 pM (Table 4), respectively.

**Table 3: HLA affinity and T cell activation time course by optimized Melanoma, Mart-1 (26-35) analogues added at 50 pM**

| Entry | Sequence | % inhibition at T=4H and T=24H | | IC₅₀ Ratio | % CD8+ T cells producing IFN-γ | | | | | Geometric mean IFN-γ in arbitrary fluorescence units | | | | |
|---|---|---|---|---|---|---|---|---|---|---|---|---|---|---|
| | | FP 4h | FP 24h | | 0H | 2H | 4H | 24H | 48H | 0H | 2H | 4H | 24H | 48H |
| 1 | [am-phg][NVA]AGIGILT[ALG] | 58 | 63 | 2 | 0 | 28 | 33 | 29 | 20 | 109 | 247 | 715 | 400 | 280 |
| 2 | [am-phg]LAGIGILT[PRG] | 68 | 65 | 7 | 0 | 22 | 31 | 28 | 18 | 105 | 249 | 480 | 342 | 215 |
| 3 | [am-phg][NVA]AGIGILT[PRG] | 67 | 64 | 8 | 0 | 28 | 32 | 28 | 18 | 110 | 252 | 550 | 344 | 222 |
| 4 | [am-phg][2-AOC]AGIGILT[PRG] | 80 | 77 | 24 | 0 | 27 | 30 | 16 | 1 | 109 | 258 | 592 | 187 | 86 |
| 5 | [CSME][2-AOC]AGIGILTV | 72 | 71 | 12 | 0 | 28 | 31 | 27 | 20 | 111 | 249 | 563 | 397 | 288 |
| 6 | ELAGIGILTV | 62 | 58 | 1 | 0 | 26 | 31 | 16 | 2 | 104 | 243 | 498 | 173 | 97 |

The 0H time point represents basal IFNγ levels of T cells in a resting state. After 2 hour incubation with peptide-MHC presenting cells, IFNγ levels have risen considerably, reaching maximum levels, as measured here at 4 hours and gradually declining at longer time points. While up to 4 hours no significant differences between index and modified peptides are apparent, at time points 24 and 48H distinct differences are found. With the exception of entry 4 all modified peptides display the ability to activate T cell for a longer duration (up to the 48 h measured) than the index peptide.

**Table 4: HLA affinity and T cell activation time course of optimized Melanoma, Mart-1 (26-35) analogues added at 0.5 pM**

| Entry | Sequence | % inhibition at T=4H and T=24H | | IC₅₀ Ratio | % CD8+ T cells producing IFN-γ | | | | | Geometric mean IFN-γ in arbitrary fluorescence units | | | | |
|---|---|---|---|---|---|---|---|---|---|---|---|---|---|---|
| | | FP 4h | FP 24h | | 0H | 2H | 4H | 24H | 48H | 0H | 2H | 4H | 24H | 48H |
| 1 | [am-phg][NVA]AGIGILT[ALG] | 58 | 63 | 2 | 0 | 26 | 29 | 17 | 5 | 112 | 221 | 350 | 163 | 111 |
| 2 | [am-phg]LAGIGILT[PRG] | 68 | 65 | 7 | 0 | 27 | 29 | 15 | 3 | 94 | 214 | 345 | 159 | 103 |
| 3 | [am-phg][NVA]AGIGILT[PRG] | 67 | 64 | 8 | 0 | 25 | 30 | 16 | 3 | 141 | 197 | 342 | 165 | 105 |
| 4 | [am-phg][2-AOC]AGIGILT[PRG] | 80 | 77 | 24 | 0 | 26 | 30 | 1 | 0 | 104 | 207 | 378 | 116 | 88 |
| 5 | [CSME][2-AOC]AGIGILTV | 72 | 71 | 12 | 0 | 27 | 29 | 18 | 7 | 101 | 219 | 368 | 167 | 119 |
| 6 | ELAGIGILTV | 62 | 58 | 1 | 0 | 19 | 20 | 1 | 0 | 117 | 181 | 230 | 102 | 92 |

Also incubation with 0.5 pM peptide shows that all modified peptides, with the exception of entry 4, are able to induce IFNγ production in responding T cells for a longer period of time than the index peptide. Moreover, as also seen with 50 pM peptide incubation, the duration of T cell activation by modified peptides is extended up to 48 hours. In contrast, at the 24 hour time point index peptide and entry 4 induce IFNγ production only just measurable above background level.

### Example 3 SVYDFFVWL - Melanoma, TRP-2, residues 180-188

This epitope stems from tyrosinase-related protein 2 (TRP-2), an enzyme expressed in most melanoma cancers. It has a moderate affinity for HLA-A2.1 making it suitable for binding enhancement. Several modified epitopes were designed and binding data as well as T cell activation data on 19 of these are plotted in **Table 5.**

Also for this epitope, an IFNγ expression time-course experiment was performed. The two distinguishing time points 24H and 48H were taken along at peptide concentrations of 5·10³ pM, 50 pM and 0.5 pM. The 5·10³ µM concentration was included because this specific T cell receptor was found to require higher peptide levels to reach the dynamic measuring range than for example the EAAGIGILTV reactive TCR discussed in Example 2.

**Table 5: HLA affinity and T cell activation (after 24 or 48 hours and at multiple peptide concentrations) by optimized Melanoma, Trp-2 (180-188) analogues**

| Entry | Sequence | % inhibition at T=4H and T=24H | | % CD8+ T cells producing IFN-γ peptide concentrations in picomolar | | | | | | Geometric mean IFN-γ in arbitrary fluorescence units peptide concentrations in picomolar | | | | | |
|---|---|---|---|---|---|---|---|---|---|---|---|---|---|---|---|
| | | | | 24H | | | 48H | | | 24H | | | 48H | | |
| | | FP 4h | FP 24h | 5·10 ³ | 50 | 0. 5 | 5·10 ³ | 50 | 0. 5 | 5·10 ³ | 50 | 0. 5 | 5·10 ³ | 5 0 | 0. 5 |
| 1 | [am-phg][NVA]YDFFVWL | 79 | 77 | 34 | 26 | 1 | 40 | 18 | 1 | 326 | 12 8 | 59 | 255 | 9 3 | 78 |
| 2 | [CSME][2-AOC]YDFFVWL | 88 | 76 | 30 | 20 | 1 | 33 | 5 | 0 | 264 | 10 9 | 71 | 224 | 7 1 | 68 |
| 3 | [PHG][NVA]YDFFVWL | 79 | 76 | 29 | 15 | 0 | 30 | 2 | 0 | 263 | 94 | 76 | 188 | 6 7 | 85 |
| 4 | [PHG][2-AOC]YDFFVWL | 90 | 75 | 30 | 14 | 0 | 33 | 3 | 1 | 224 | 95 | 85 | 184 | 6 8 | 75 |
| 5 | [PHG][CpALA]YDFFVWL | 77 | 75 | 30 | 12 | 1 | 34 | 6 | 0 | 221 | 87 | 71 | 183 | 7 0 | 70 |
| 6 | [am-phg][2-AOC]YDFFVWL | 94 | 87 | 31 | 12 | 0 | 30 | 1 | 0 | 215 | 82 | 78 | 143 | 70 | 88 |
| 7 | [PHG][NVA]YDFFVW[ALG] | 76 | 73 | 35 | 6 | 0 | 22 | 1 | 1 | 185 | 71 | 84 | 142 | 64 | 64 |
| 8 | [PHG][NVA]YDFFVW[PRG] | 83 | 78 | 33 | 6 | 1 | 21 | 1 | 0 | 183 | 73 | 89 | 139 | 61 | 66 |
| 9 | [am-phg][NVA]YDFFVW[PRG] | 88 | 80 | 26 | 7 | 0 | 23 | 1 | 0 | 181 | 77 | 64 | 132 | 57 | 71 |
| 10 | [CSME][NVA]YDFFVW[PRG] | 78 | 78 | 26 | 6 | 0 | 26 | 1 | 1 | 175 | 80 | 67 | 130 | 67 | 93 |
| 11 | [PHG][CpALA]YDFFVW[PRG] | 87 | 82 | 28 | 6 | 0 | 18 | 1 | 1 | 168 | 72 | 63 | 116 | 57 | 67 |
| 12 | [CSME][CpALA]YDFFVW[PRG] | 85 | 83 | 27 | 5 | 1 | 14 | 1 | 0 | 147 | 73 | 69 | 105 | 68 | 58 |
| 13 | [PHG][2-AOC]YDFFVW[ALG] | 75 | 72 | 25 | 2 | 0 | 14 | 0 | 0 | 145 | 68 | 72 | 96 | 69 | 80 |
| 14 | [PHG][2-AOC]YDFFVW[PRG] | 83 | 78 | 20 | 1 | 0 | 0 | 0 | 1 | 120 | 68 | 95 | 78 | 78 | 79 |
| 15 | [CSME][2-AOC]YDFFVW[ALG] | 81 | 81 | 21 | 1 | 0 | 0 | 0 | 0 | 119 | 59 | 54 | 78 | 68 | 61 |
| 16 | [am-phg][CpALA]YDFFVW[PRG] | 80 | 80 | 14 | 1 | 1 | 4 | 0 | 0 | 95 | 69 | 94 | 76 | 66 | 76 |
| 17 | [CSME][2-AOC]YDFFVW[PRG] | 98 | 89 | 7 | 1 | 0 | 0 | 0 | 0 | 84 | 73 | 67 | 72 | 6 | 67 |
| 18 | [am-phg][2-AOC]YDFFVW[ALG] | 73 | 73 | 7 | 1 | 0 | 4 | 0 | 0 | 79 | 69 | 98 | 71 | 80 | 76 |
| 19 | [am-phg][2-AOC]YDFFVW[PRG] | 87 | 84 | 1 | 1 | 0 | 1 | 1 | 1 | 65 | 65 | 64 | 64 | 89 | 63 |
| 20 | SVYDFFVWL | 61 | 62 | 24 | 2 | 0 | 6 | 0 | 0 | 140 | 64 | 72 | 91 | 64 | 59 |

The results support the findings obtained for the Mart-1 ELAGIGILTV epitope. When enhanced by incorporating unnatural residues, the derivatives gain the ability to induce IFNγ expression for a longer period of time. Already at timepoint 24H a difference is observed at the 5·10³ pM concentration when comparing the natural epitope to the better performing derivatives. Even more so this same effect is apparent after 48H.

At 50 pM and 24H the natural epitope induced IFNγ expression has fallen to background level, whereas the enhanced epitopes still show significant expression of this cytokine. At the same concentration but after 48H, only entries 1, 2 and 6 still show IFNγ expression significantly above background level. For the epitope-TCR combination studied here, incubation with 0.5 pM peptide does not lead to T cell activation in all cases.

### Example 4 [SOME] substitution at position P₁

HLA affinity is represented by percentage inhibition scores obtained using a fluorescence polarization assay as described in the materials and methods section. As shown in **Table 6,** HLA affinity of wild-type peptides GILGFVFTL and EAAGIGILTV can be improved, as indicated by the percentage of inhibition by [SOME] replacements at position P₁ resulting in [SOME]ILGFVFTL and [SOME]AAGIGILTV.

**Table 6 HLA affinity of optimised epitopes containing [SOME] substitutions at position P₁**

| Entry | Sequence | % inhibition at T=4H and T=24H | |
|---|---|---|---|
| | | FP 4H | FP 24H |
| 1 | GILGFVFTL | 81 | 78 |
| 2 | [SOME]ILGFVFTL | 85 | 83 |
| 3 | EAAGIGILTV | 51 | 18 |
| 4 | ELAGIGILTV | 62 | 58 |
| 5 | [SOME]AAGIGILTV | 63 | 60 |

### Example 5 [3-THI] substitutions at position P₁

HLA affinity is represented by percentage inhibition scores obtained using a fluorescence polarization assay as described in the materials and methods section. As shown in **Table 7,** wild-type peptide QLLNSVLTV was modified by [3-THI] replacement at position P₁ resulting in [3-THI]LLNSVLTV, [3-THI]LLNSVLT[2-AOC], [3-THI][2-AOC]LNSVLT[NVA] and [3-THI][NLE]LNSVLT[NVA].

T cells specific for the epitope QLLNSVLTV were stimulated with several concentrations of this epitope, the modified peptide [3-THI]LLNSVLTV, or three similar 3-THI containing peptides. Interferon-γ production, indicative of T cell activation, was measured by an ELISA.

**Table 7 T cell activation by epitopes containing [3-THI] substitution at position P₁ as determined by an interferon-γ production ELISA.***

| | | Concentration of peptide in nanomolars | | | | |
|---|---|---|---|---|---|---|
| Entry | Sequence | 0.1 | 1 | 10 | 100 | 1000 |
| | | | | | | |
| 1 | [3-THI]LLNSVLTV | 1182 | 4515 | 5000 | 5000 | 5000 |
| | | | | | | |
| 2 | [3-THI]LLNSVLT[2-AOC] | 625 | 1251 | 5000 | 5000 | 5000 |
| 3 | [3-THI][2-AOC]LNSVLT[NVA] | 454 | 790 | 1197 | 3395 | 2904 |
| 4 | [3-THI][NLE]LNSVLT[NVA] | 1140 | 1726 | 3373 | 2887 | 2536 |
| | | | | | | |
| 5 | QLLNSVLTL | 1156 | 1865 | 1548 | 2714 | 5000 |
| 6 | QLLNSVLTL | 1359 | 3007 | 5000 | 5000 | 3664 |
| 7 | QLLNSVLTL | 588 | 2806 | 5000 | 4174 | 5000 |

| | | | | | | |
|---|---|---|---|---|---|---|
| ^{*)}Values obtained are arbitrary fluorescence units. | | | | | | |

It is observed that the 3-THI residue has a positive effect on T cell activation, especially in combination with a leucine to valine replacement on position 9 of the peptide (entry 1). Entries 2, 3 and 4 show that the 3-THI residue is well tolerated on P₁, even when combined with multiple other substitutions. The wild-type epitope was taken along three times (entries 5, 6 and 7).

### Example 6 [CSCF3] substitution at position P₁

HLA affinity is represented by percentage inhibition scores obtained using a fluorescence polarization assay as described in the materials and methods section. As shown in **Table 8,** the binding of wild-type peptides ELAGIGILTV and SVYDFFVWL to HLA A2 can be improved, as indicated by the percentage of inhibition, by [CSCF3] replacement at position P₁ resulting in [CSCF3][2-AOC]AGIGILTV and [CSCF3][2-AOC]YDFFVWL.

**Table 8 HLA affinity of epitopes containing a [CSCF3] substitution at position P₁**

| Entry | Sequence | % inhibition at T=4H and T=24H | |
|---|---|---|---|
| | | FP 4H | FP 24H |
| 1 | ELAGIGILTV | 62 | 58 |
| 2 | [CSME][2-AOC]AGIGILTV | 72 | 71 |
| 3 | [CSCF3][2-AOC]AGIGILTV | 86 | 86 |
| | | | |
| 4 | SVYDFFVWL | 61 | 62 |
| 5 | [CSME][2-AOC]YDFFVWL | 88 | 76 |
| 6 | [CSCF3][2-AOC]YDFFVWL | 91 | 79 |

The binding of both wild-type peptides to HLA A2 (entries 1 and 4 in the table) can be improved by replacement of P₁ and P₂ with [CSME] [2-AOC] (entries 2 and 5). These can be further improved by a [CSME] to [CSCF3] replacement on P₁ (entries 3 and 6).

### Example 7 [3F-ABU] substitution at position P₂

HLA affinity is represented by percentage inhibition scores obtained using a fluorescence polarization assay as described in the materials and methods section. As shown in **Table 9,** the binding of wild-type peptide EAAGIGILTV to HLA A2 can be improved, as indicated by the percentage of inhibition, by [3F-ABU] replacement at position P₂ resulting in E[3F-ABU]AGIGILTV.

**Table 9 HLA affinity of epitopes containing a [3F-ABU] substitution at position P₂**

| Entry | Sequence | % inhibition at T=4H and T=24H | |
|---|---|---|---|
| | | FP 4H | FP 24H |
| 1 | EAAGIGILTV | 51 | 18 |
| 2 | E[3F-ABU]AGIGILTV | 50 | 49 |

Replacement of the alanine on P₂ of the wild-type epitope with a racemic mixture of 3F-ABU residue increases binding at the 24H time point. It was further found that the enantiopure L-form of [3F-ABU] increased binding even further.

### Example 8 In vivo vaccination studies

Three mouse vaccination studies were done using three different wild-type epitopes. In all cases, mice were vaccinated with the peptide supplemented with incomplete freunds ajuvant (IFA) and CpG. Analysis was done by analyzing blood samples with tetramers loaded with wild-type peptide in all cases.

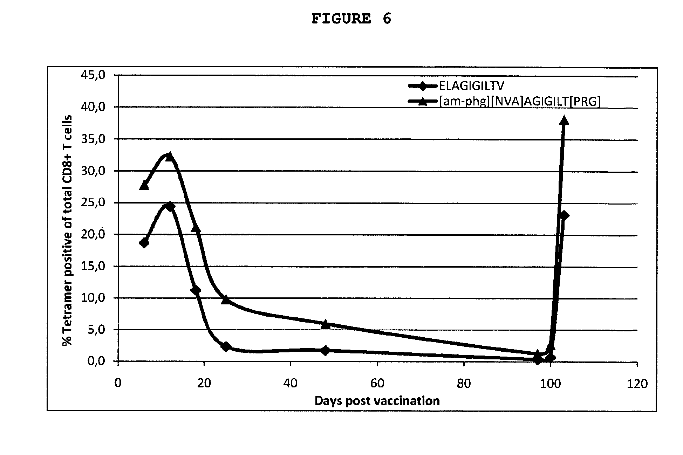

Two groups of three mice were vaccinated with 5 µg of either ELAGIGILTV or [am-phg][NVA]AGIGILT[PRG]. At day 97, mice were administred a second dose (booster). At several timepoints blood was taken and tested for ELAGIGILTV-specific CD8+ T cells using tetramers loaded with ELAGIGILTV. This was done for both groups of mice. At all times, specific T cell numbers were higher for the [am-phg][NVA]AGIGILT[PRG] vaccinated mice. The results are summarised in **Figure 6****.**

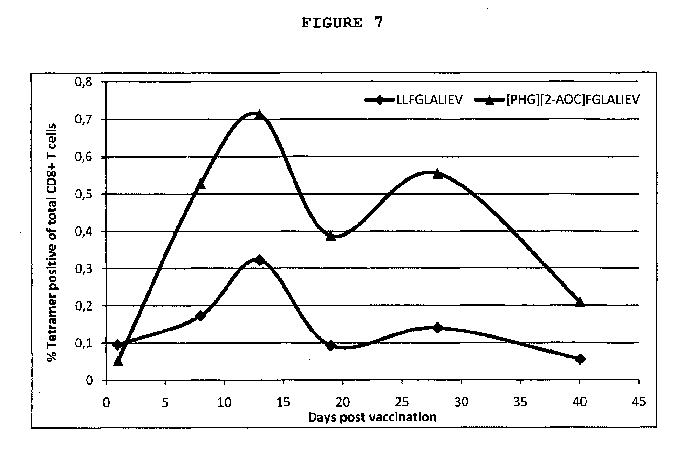

Two groups of four mice were vaccinated with with 100 µg of either LLFGLALIEV or [PHG][2-AOC]FGLALIEV. LLFGLALIEV is an immunogenic peptide derived from Melanoma-associated protein C2 (Mage-C2). At several timepoints blood was taken and tested for LLFGLALIEV-specific CD8+ T cells using tetramers loaded with LLFGLALIEV. This was done for both groups of mice. At all times after day one, specific T cell numbers were higher for the [PHG][2-AOC]FGLALIEV vaccinated mice. The results are summarised in **Figure 7****.**

These peptides were also tested for HLA binding and T cell activation using an existing T cell line specific for LLFGLALIEV **(Table 10).** HLA binding is represented by percentage inhibition scores were obtained using a fluorescence polarization assay as described in the materials and methods section. T cell activation was quantified using the FACS based interferon-gamma production assay as materials and methods section.

**Table 10 HLA binding and T cell activation of modified Melanoma-associated protein C2 (pp-qq) epitopes used in vaccination studies.**

| Entry | Sequence | % inhibition at T=4H and T=24H | | % Positive TCR recognition | Geometric mean in arbitrary fluorescence units |
|---|---|---|---|---|---|
| | | FP 4h | FP 24h | % TCR | Geo TCR |
| 1 | LLFGLALIEV | 50 | 47 | 11.5 | 108 |
| 2 | [PHG][2-AOC]FGLALIEV | 81 | 81 | 13.7 | 108 |

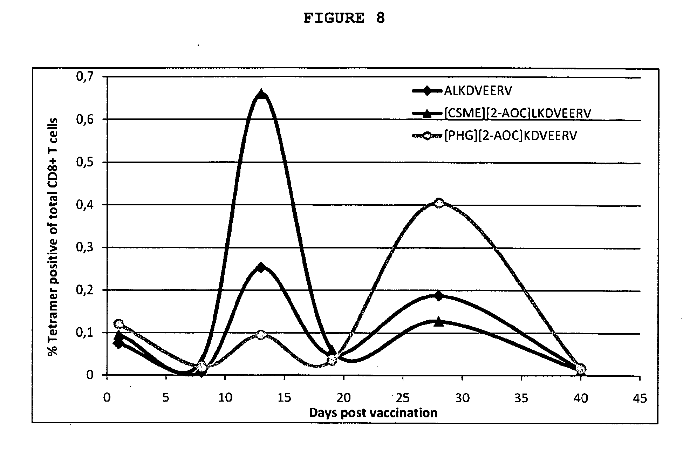

Three groups of four mice were vaccinated with with 100 µg of either ALKDVEERV, [PHG][2-AOC]KDVEERV or [CSME][2-AOC]KDVEERV. ALKDVEERV is an immunogenic peptide derived from Melanoma-associated protein C2 (Mage-C2). At several time points blood was taken and tested for ALKDVEERV -specific CD8+ T cells using tetramers loaded with ALKDVEERV. This was done for all three groups of mice. [CSME][2-AOC]KDVEERV gave the highest initial response whereas [PHG][2-AOC]KDVEERV came up later but still gave a higher response than the wild-type ALKDVEERV peptide did. The results are summarised in **Figure 8****.**

These peptides were also tested for MHC binding and T cell response using an existing T cell line specific for ALKDVEERV **(Table 11).** Percentage inhibition scores were obtained using a fluorescence polarization assay as described. T cell assay results were obtained following the protocol described in the materials and methods section.

**Table 11 HLA binding and T cell responseactivation of modified Melanoma-associated protein C2(xx-yy) epitopes used in vaccination studies**

| Entry | Sequence | % inhibition at T=4H and T=24H | | % Positive TCR recognition | Geometric mean in arbitrary fluorescence units |
|---|---|---|---|---|---|
| | | FP 4h | FP 24h | % TCR | Geo TCR |
| 1 | ALKDVEERV | 27 | 21 | 42.2 | 379 |
| 2 | [PHG][CSME]KDVEER V | 39 | 31 | 37.8 | 352 |
| 3 | [CSME][2-AOC]KDVEERV | 60 | 56 | 36.7 | 327 |

### Example 9 Various amino acid substitutions

Similar to examples 1 to 7, various amino acid substitutions in peptides EAAGIGILTV, FMYSDFHFI and VIWEVLNAV were explored the results of which are summarised in **Table 12** below.

**Table 12 Amino acid substitutions in peptides EAAGIGILTV, FMYSDFHFI and VIWEVLNAV**

| Entry | Sequence | % inhibition at T=4H and T=24H | |
|---|---|---|---|
| | | FP 4H | FP 24H |
| 1 | EAAGIGILTV | 51 | 18 |
| 2 | [3-PYRA]AAGIGILTV | 50 | 47 |
| 3 | GILGFCFTL | 81 | 78 |
| 4 | [3-PYRA]ILGFVFTL | 87 | 85 |
| 5 | GLCTLVAML | 56 | 43 |
| 6 | [3-PYRA]LCTLVAML | 75 | 72 |
| 7 | YLEPGPVTA | 63 | 40 |
| 8 | [3-PYRA]LEPGPVTA | 50 | 47 |
| 9 | FMYSDFHFI | 75 | 74 |
| 10 | [3-PYRA]MYSDFHFI | 79 | 76 |
| | | | |
| 11 | [3-PYRA][2-AOC]WEVLNA[CSME] | 102 | 102 |
| 12 | [4-FPHE][2-AOC]WEVLNA[CSME] | 92 | 87 |
| 13 | [PRG][2-AOC][NLE]EVLNA[CSME] | 87 | 84 |
| 14 | [3-PYRA][NLE]WEVLNA[CSME] | 81 | 79 |
| 15 | [3-PYRA][ALG]WEVLNA[CSME] | 80 | 79 |
| 16 | V[2-AOC]WEVL[BUTGLY]A[CSME] | 72 | 70 |
| 17 | [PHG][NLE]WEVLNA[CSME] | 73 | 70 |
| 18 | [3-PYRA][OM-HS]WEVLNA[CSME] | 72 | 67 |
| 19 | [PRG][NLE]WEVLNA[CSME] | 70 | 66 |
| 20 | [PHG][ALG]WEVLNA[CSME] | 68 | 65 |
| 21 | [PRG][ALG]WEVLNA[CSME] | 59 | 48 |
| 22 | VIWEVLNAV | 50 | 44 |

### Conclusion

In summary, the present examples show that the unnatural peptide analogues, containing non-naturally occurring amino acids, display stronger MHC binding and show stronger and prolonged capacity to induce T cell activation at concentrations lower than required for their natural counterparts.

## Claims

1. Immune restricted peptide, preferably an HLA-A2 immune restricted peptide, according to the general formula (I) : 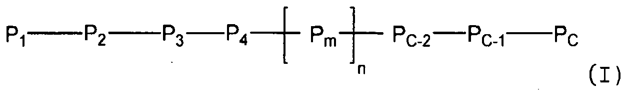 wherein:
- P₁ is selected from the group consisting of am-phg, PHG, 3-PYRA, 3-THI, SOME, CSCF3 and CSME;
- P₂ is an amino acid comprising a hydrophobic linear or branched or fluorinated substitution;
- P₃ is an amino acid comprising a hydrophobic linear or branched substitution;
- P₄ is an amino acid comprising a methylated alpha nitrogen atom;
- Pₘ is a naturally occurring amino acid, n is an integer of 1 to 9, preferably 1 to 4, more preferably 2 or 3;
- P_{C-2} is an amino acid comprising a fluorinated aromatic substitution;
- P_{C-1} is an amino acid;
- P_{C} is an amino acid comprising unsaturated or saturated side chains and/or carboxyl isosteres or a saturated linear carbon chain containing 2 to 4 carbon atoms with or without an oxygen atom or a sulphur atom within the chain and/or a carboxyl isostere.

2. Immune restricted peptide according to claim 1,
wherein further at least two, preferably at least 3, more preferably at least 4, most preferably 5, of P₂, P₃, P₄, P_{C-2} and P_{C} are a non-naturally occurring amino acid.

3. Immune restricted peptide according to claim 1 or claim 2, wherein: 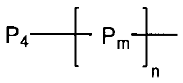 is defined as the residues that make up the bulk part of the interaction site between peptide and TCR, and is preferably a fragment of an HLA-A2 restricted immunogenic epitope.

4. Immune restricted peptide according to any of the claims 1 to 3 according to the general formula (II): 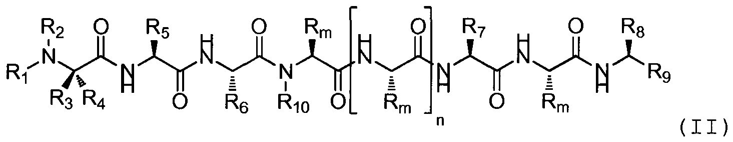 wherein:
- R₁ to R₄ are as defined by am-phg, PHG, 3-PYRA, 3-THI, SOME, CSCF3 or CSME;
- R₅ is a fluorinated or non-fluorinated saturated linear aliphatic chain containing 2 to 6 carbon atoms with or without an oxygen atom or a sulfur atom within the chain or is defined as in 3F-ABU;
- R₆ is a saturated linear aliphatic chain containing 2 to 6 carbon atoms; and/or
- R₇ is a fluorinated benzyl moiety; and/or
- R₈ is an unsaturated carbon chain comprising of 2 to 3 carbon atoms and R₉ is carboxyl; and/or
- R₈ is a saturated or unsaturated linear or branched carbon chain containing 2 to 4 carbon atoms with or without an oxygen atom or a sulphur atom within the chain, or a terminal thiol group and R₉ is selected from the group consisting of carboxylate, tetrazole, boronate, phosphonate, N-acyl-S-alkyl-sulfonamides (specifically N-acyl-S-methyl-sulfonamide), and hydroxamate; and/or
- R₁₀ is H or methyl; and/or
- Rₘ is a naturally occurring amino acid side chain.

5. Immune restricted peptide according to any of the claims 1 to 4, wherein P_{C-2} is 4-FPHE.

6. Immune restricted peptide according to any of the claims 1 to 5, wherein P₂ is selected from the group consisting of CₚALA, NLE, BUTALA, NVA, 3F-ABU, (L)3F-ABU and 2-AOC.

7. Immune restricted peptide according to any of the claims 1 to 6, wherein P₃ is NLE.

8. Immune restricted peptide according to any of the claims 1 to 7, wherein P₄ is an alpha N-methylated amino acid.

9. Immune restricted peptide according to any of the claims 1 to 8, wherein P_{C} is selected from the group consisting of ALG, PRG, NLE, CSME and OM-HS.

10. Immune restricted peptide according to any of the claims 1 to 9, wherein 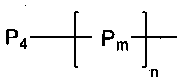 is selected from the group GFV, GIGI or DFF.

11. Method for providing an immune restricted peptide, preferably an HLA-A2 immune restricted peptide, comprising:
a) selecting an immunogenic peptide, preferably an HLA-A2 immunogenic peptide, represented by the formula 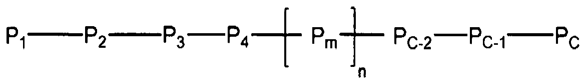 wherein P₁, P₂, P₃, P₄, Pₘ, P_{C-2} P_{C-1} and P_{C} are naturally occurring amino acids;
b) replacing at least one of the naturally occurring amino acids at position P₁ and, preferably, at least one of the positions P₂, P₃, P₄, P_{C-2} and P_{C} by a non-naturally occurring amino acid as defined in any of the claims 1 to 10 thereby providing an immune restricted peptide, preferably an HLA-A2 restricted peptide
wherein the replacement of P₁ is selected from the group consisting of am-phg, PHG, 3-PYRA, 3-THI, SOME, CSCF3 and CSME.

12. Immune restricted peptide according to any of the claims 1 to 10 for use as a vaccine, for use in immunosuppressive therapy or T cell antagonism or for use in adoptive T cell therapy.

## Patentansprüche

1. Immunbeschränktes Peptid, bevorzugt ein HLA-A2-immunbeschränktes Peptid, gemäß der allgemeinen Formel (I): 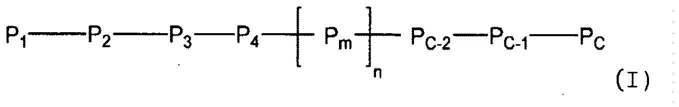 wobei:
- P₁ ausgewählt ist aus der Gruppe bestehend aus am-phg, PHG, 3-PYRA, 3-THI, SOME, CSCF3 und CSME;
- P₂ eine Aminosäure ist, die eine hydrophobe lineare oder verzweigte oder fluorierte Substitution umfasst;
- P₃ eine Aminosäure ist, die eine hydrophobe lineare oder verzweigte Substitution umfasst;
- P₄ eine Aminosäure ist, die ein methyliertes alpha-Stickstoffatom umfasst;
- Pₘ eine natürlich auftretende Aminosäure ist, n eine ganze Zahl von 1 bis 9, bevorzugt 1 bis 4, stärker bevorzugt 2 oder 3 ist;
- P_{C-2} eine Aminosäure ist, die eine fluorierte aromatische Substitution umfasst;
- P_{C-1} eine Aminosäure ist;
- P_{C} eine Aminosäure ist, die ungesättigte oder gesättigte Seitenketten und/ oder Carboxylisostere oder eine gesättigte lineare Kohlenstoffkette mit 2 bis 4 Kohlenstoffatomen mit oder ohne ein Sauerstoffatom oder ein Schwefelatom innerhalb der Kette und/oder ein Carboxylisoster umfasst.

2. Immunbeschränktes Peptid gemäß Anspruch 1, wobei weiterhin mindestens zwei, bevorzugt mindestens drei, stärker bevorzugt mindestens vier, am stärksten bevorzugt fünf von P₂, P₃, P₄, P_{C-2} und P_{C} nicht natürlich auftretende Aminosäuren sind.

3. Immunbeschränktes Peptid gemäß Anspruch 1 oder 2, wobei: 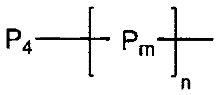 als die Reste definiert ist, die den Großteil der Wechselwirkungsstelle zwischen Peptid und TCR ausmachen, und bevorzugt ein Fragment eines HLA-A2-beschränkten immunogenen Epitops ist.

4. Immunbeschränktes Peptid gemäß einem der Ansprüche 1 bis 3 gemäß der allgemeinen Formel (II): 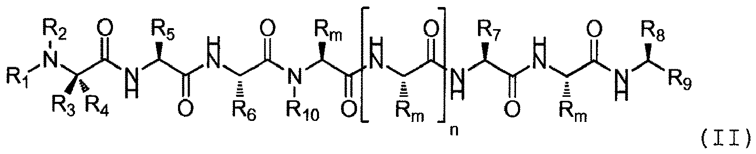 wobei:
- R₁ bis R₄ definiert sind durch am-phg, PHG, 3-PYRA, 3-THI, SOME, CSCF3 oder CSME;
- R₅ eine fluorierte oder nicht fluorierte gesättigte lineare aliphatische Kette ist, die 2 bis 6 Kohlenstoffatome mit oder ohne ein Sauerstoffatom oder ein Schwefelatom innerhalb der Kette enthält oder wie in 3F-ABU definiert ist;
- R₆ eine gesättigte lineare aliphatische Kette ist, die 2 bis 6 Kohlenstoffatome enthält; und/oder
- R₇ ein fluorierter Benzylrest ist; und/oder
- R₈ eine ungesättigte Kohlenstoffkette ist, die 2 bis 3 Kohlenstoffatome enthält und R₉ Carboxyl ist; und/oder
- R₈ eine gesättigte oder ungesättigte lineare oder verzweigte Kohlenstoffkette mit 2 bis 4 Kohlenstoffatomen mit oder ohne ein Sauerstoffatom oder ein Schwefelatom innerhalb der Kette oder eine endständige Thiolgruppe ist und R₉ ausgewählt ist aus der Gruppe bestehend aus Carboxylat, Tetrazol, Boronat, Phosphonat, N-Acyl-S-Alkylsulfonamiden (besonders N-Acyl-S-Methylsulfonamid) und Hydroxamat; und/oder
- R₁₀ H oder Methyl ist; und/oder
- Rₘ eine natürlich auftretende Aminosäureseitenkette ist.

5. Immunbeschränktes Peptid gemäß einem der Ansprüche 1 bis 4, wobei P_{C-2} 4-FPHE ist.

6. Immunbeschränktes Peptid gemäß einem der Ansprüche 1 bis 5, wobei P₂ ausgewählt ist aus der Gruppe bestehend aus CₚALA, NLE, BUTALA, NVA, 3F-ABU, (L)3F-ABU und 2-AOC.

7. Immunbeschränktes Peptid gemäß einem der Ansprüche 1 bis 6, wobei P₃ NLE ist.

8. Immunbeschränktes Peptid gemäß einem der Ansprüche 1 bis 7, wobei P₄ eine alpha N-methylierte Aminosäure ist.

9. Immunbeschränktes Peptid gemäß einem der Ansprüche 1 bis 8, wobei P_{C} ausgewählt ist aus der Gruppe bestehend aus ALG, PRG, NLE, CSME und OM-HS.

10. Immunbeschränktes Peptid gemäß einem der Ansprüche 1 bis 9, wobei 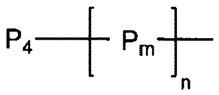 ausgewählt ist aus der Gruppe GFV, GIGI oder DFF.

11. Verfahren zum Bereitstellen eines immunbeschränkten Peptids, bevorzugt eines HLA-A2-immunbeschränkten Peptids, das Folgendes umfasst:
a) Auswählen eines immunogenen Peptids, bevorzugt eines HLA-A2-immunogenen Peptids, dargestellt durch die Formel 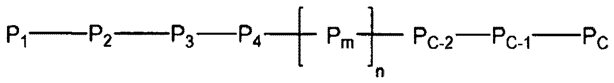 wobei P₁, P₂, P₃, P₄, Pₘ, P_{C-2}, P_{C-1} und P_{C} natürlich auftretende Aminosäuren sind;
b) Ersetzen mindestens einer der natürlich auftretenden Aminosäuren an Position P₁ und, bevorzugt, mindestens einer der Positionen P₂, P₃, P₄, P_{C-2} und P_{C} durch eine nicht natürlich auftretende Aminosäure wie in einem der Ansprüche 1 bis 10 definiert, so dass ein immunbeschränktes Peptid, bevorzugt ein HLA-A2-beschränktes Peptid bereitgestellt wird,
wobei der Ersatz von P₁ ausgewählt ist aus der Gruppe bestehend aus am-phg, PHG, 3-PYRA, 3-THI, SOME, CSCF3 und CSME.

12. Immunbeschränktes Peptid gemäß einem der Ansprüche 1 bis 10 zur Verwendung als Impfstoff, zur Verwendung bei immunsuppressiver Therapie oder T-Zellenantagonismus oder zur Verwendung bei adoptiver T-Zellentherapie.

## Revendications

1. Peptide immunorestreint, de préférence peptide immunorestreint à HLA-A2 selon la formule générale (I) : 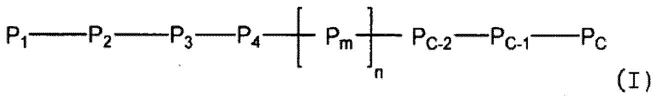 dans laquelle
- P₁ est sélectionné dans le groupe constitué d'am-phg, de PHG, de 3-PYRA, de 3-THI, de SOME, de CSCF3 et de CSME ;
- P₂ est un acide aminé comprenant une substitution hydrophobe linéaire ou ramifiée ou fluorée ;
- P₃ est un acide aminé comprenant une substitution hydrophobe linéaire ou ramifiée ;
- P₄ est un acide aminé comprenant un atome d'azote alpha méthylé,
- Pₘ est un acide aminé existant à l'état naturel, n est un nombre entier de 1 à 9, de préférence de 1 à 4, de manière davantage préférée de 2 ou 3 ;
- P_{C-2} est un acide aminé comprenant une substitution aromatique fluorée ;
- P_{c-1} est un acide aminé ;
- P_{c} est un acide aminé comprenant des chaînes latérales insaturées ou saturées et/ou des isostères carboxyle ou une chaîne de carbone linéaire saturée contenant 2 à 4 atomes de carbone avec ou sans atome d'oxygène ou un atome de soufre dans la chaîne et/ou un isostère carboxyle.

2. Peptide immunorestreint selon la revendication 1, dans lequel en outre au moins deux, de préférence au moins 3, de manière davantage préférée au moins 4, de manière référée entre toutes au moins 5 de P₂, P₃, P₄, P_{c-2} et P_{c} sont un acide aminé non présent à l'état naturel.

3. Peptide immunorestreint selon la revendication 1 ou la revendication 2, dans lequel : 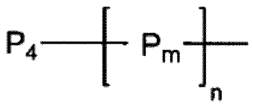 est défini comme les résidus qui constituent la majeure partie du site d'interaction entre peptide et TCR et est de préférence un fragment d'un épitope immunogène restreint HLA-A2.

4. Peptide immunorestreint selon l'une quelconque des revendications 1 à 3, selon la formule générale (II) : 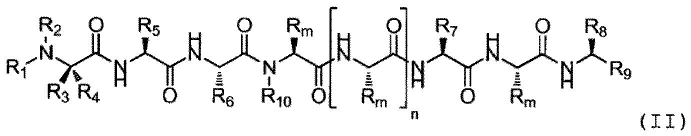 dans laquelle
- R₁ à R₄ sont tel que définis par am-phg, PHG, 3-PYRA, 3-THI, SOME, CSCF3 ou CSME;
- R₅ est une chaîne aliphatique linéaire saturée fluorée ou non fluorée contenant 2 à 6 atomes de carbone avec ou sans atome d'oxygène ou atome de soufre dans la chaîne ou est défini comme dans 3F-ABU ;
- R₆ est une chaîne aliphatique linéaire saturée contenant 2 à 6 atomes de carbone ; et/ou
- R₇ est une fraction benzyle fluorée ; et/ou
- R₈ est une chaîne de carbone insaturée comprenant 2 à 3 atomes de carbone et R₉ est un groupe carboxyle ; et/ou
- R₈ est une chaîne de carbone linéaire ou ramifiée saturée ou insaturée contenant 2 à 4 atomes de carbone avec ou sans atome d'oxygène ou atome de soufre dans la chaîne, ou un groupe thiol terminal et R₉ est sélectionné dans le groupe constitué d'un carboxylate, d'un tétrazole, d'un boronate, d'un phosphonate, de N-acyl-S-alkyl-sulfonamides (en particulier, N-acyl-S-méthyl-sulfonamide) et d'un hydroxamate ; et/ou
- R₁₀ est H ou un groupe méthyle ; et/ou
- Rₘ est une chaîne latérale d'un acide aminé existant à l'état naturel.

5. Peptide immunorestreint selon l'une quelconque des revendications 1 à 4, dans lequel P_{c-2} est le 4-FPHE.

6. Peptide immunorestreint selon l'une quelconque des revendications 1 à 5, dans lequel P₂ est sélectionné dans le groupe constitué de CₚALA, NLE, BUTALA, NVA, 3F-ABU, (L)3F-ABU et 2-AOC.

7. Peptide immunorestreint selon l'une quelconque des revendications 1 à 6, dans lequel P₃ est NLE.

8. Peptide immunorestreint selon l'une quelconque des revendications 1 à 7, dans lequel P₄ est un acide aminé alpha N-méthylé.

9. Peptide immunorestreint selon l'une quelconque des revendications 1 à 8, dans lequel P_{c} est sélectionné dans le groupe constitué d'ALG, de PRG, de NLE, de CSME et d'OM-HS.

10. Peptide immunorestreint selon l'une quelconque des revendications 1 à 9, dans lequel 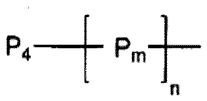 est sélectionné dans le groupe GFV, GIGI ou DFF.

11. Procédé pour fournir un peptide immunorestreint, de préférence un peptide immunorestreint HLA-A2 comprenant :
a) la sélection d'un peptide immunogène, de préférence un peptide immunogène HLA-A2, représenté par la formule 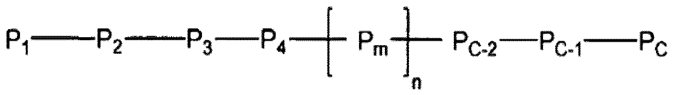 dans laquelle P₁, P₂, P₃, P₄, Pₘ, P_{c-2} et P_{c} sont des acides aminés existants à l'état naturel ;
b) le remplacement d'au moins l'un des acides aminés existant à l'état naturel en position P₁ et, de préférence, au moins l'une des positions P₂, P₃, P₄, P_{c-2} et P_{c} par un acide aminé non existant à l'état naturel tel que défini dans l'une quelconque des revendications 1 à 10, fournissant ainsi un peptide immunorestreint, de préférence, un peptide à HLA-A2 restreint dans lequel le remplacement de P₁ est sélectionné dans le groupe constitué d'am-phg, de PHG, de 3-PYRA, de 3-THI, de SOME, de CSCF3 et de CSME.

12. Peptide immunorestreint selon l'une quelconque des revendications 1 à 10, pour son utilisation comme vaccin, pour son utilisation dans un traitement immunosuppresseur ou des antagonismes des lymphocytes T ou pour son utilisation dans un traitement adoptif de lymphocytes T.
